(19) Europäisches Patentamt
European Patent Office
Office européen des brevets

(11) EP 3 851 850 A1

(12) # EUROPEAN PATENT APPLICATION
published in accordance with Art. 153(4) EPC

(43) Date of publication:
**21.07.2021 Bulletin 2021/29**

(21) Application number: **19860272.4**

(22) Date of filing: **05.09.2019**

(51) Int Cl.:
***G01N 30/06*** *(2006.01)* ***G01N 30/88*** *(2006.01)*

(86) International application number:
**PCT/JP2019/034972**

(87) International publication number:
**WO 2020/054572 (19.03.2020 Gazette 2020/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **12.09.2018 JP 2018170562**
**12.09.2018 JP 2018170563**

(71) Applicants:
- **Sekisui Medical Co., Ltd.**
  **Tokyo 103-0027 (JP)**
- **Tokuyama Sekisui Co., Ltd.**
  **Osaka-Shi, Osaka 530-8565 (JP)**

(72) Inventors:
- **OISHI, Kazuyuki**
  **Tokyo 103-0027 (JP)**
- **TAIRA, Hiroaki**
  **Tokyo 103-0027 (JP)**
- **INOUE, Tomonori**
  **Shunan-shi, Yamaguchi 746-0006 (JP)**
- **TAKEMOTO, Ryo**
  **Shunan-shi, Yamaguchi 746-0006 (JP)**

(74) Representative: **Ter Meer Steinmeister & Partner**
**Patentanwälte mbB**
**Nymphenburger Straße 4**
**80335 München (DE)**

(54) **REAGENT AND METHOD FOR MEASURING HEMOGLOBINS**

(57) Provided is a reagent for measuring hemoglobins capable of satisfactorily causing hemolysis and measuring hemoglobins with high accuracy. The reagent for measuring hemoglobins according to the present invention is a reagent for measuring hemoglobins used for measuring hemoglobins by cation exchange liquid chromatography, including a specific nonionic surfactant or a specific amphoteric surfactant.

EP 3 851 850 A1

## Description

### TECHNICAL FIELD

The present invention relates to a reagent for measuring hemoglobins including a surfactant. The present invention also relates to a method for measuring hemoglobins using the reagent for measuring hemoglobins.

### BACKGROUND ART

Conventionally, the concentration of hemoglobins is measured for the diagnosis of diabetes, hemoglobinopathy and the like. For example, the concentration of hemoglobins including normal hemoglobins such as hemoglobin A1a, hemoglobin A1b, hemoglobin F, hemoglobin A1c, hemoglobin A0, and hemoglobin A2, modified hemoglobins such as acetylated hemoglobin and carbamylated hemoglobin, and abnormal hemoglobins such as hemoglobin S and hemoglobin C is measured. Among these hemoglobins, in particular, the value of hemoglobin A1c is measured. Examples of the method for measuring the concentration of hemoglobins mainly includes a liquid chromatography method.

In the liquid chromatography method, a hemolytic reagent is added to a sample such as blood, the sample after hemolysis is measured by cation exchange liquid chromatography, and the peak of the obtained chromatogram is arithmetically processed to determine the concentration of hemoglobins such as the value of hemoglobin A1c. Conventionally, as a hemolytic reagent, a composition containing polyoxyethylene octyl phenyl ether having an average number of moles of an oxyethylene group added of 10 (for example, trade name "Triton X-100") is used.

Patent Document 1 below discloses a method for separating hemoglobin A1c for separating hemoglobin A1c in a sample of human blood from other glycosylated and non-glycosylated hemoglobins and a Schiff base of a precursor of hemoglobin A1c. In Example of Patent Document 1, a hemolytic reagent containing 0.33% by weight of Triton X-100 is used.

Patent Document 2 below discloses a hemolytic reagent used for hemolyzing a blood sample in a method for measuring hemoglobins by cation exchange liquid chromatography. The hemolytic reagent contains chaotropic ions. In Example of Patent Document 2, a hemolytic reagent containing 0.1% by weight of Triton X-100 is used.

### Related Art Document

### Patent Documents

Patent Document 1: JP S58-191968 A
Patent Document 2: JP 2001-021555 A

### SUMMARY OF THE INVENTION

### PROBLEMS TO BE SOLVED BY THE INVENTION

Hemolytic reagents (reagents for measuring hemoglobins) including polyoxyethylene octyl phenyl ether having an average number of moles of an oxyethylene group added of 10 as described in Patent Documents 1 and 2 are excellent in hemolytic performance. Thus, when a sample such as whole blood is treated with such a hemolytic reagent and is measured by cation exchange liquid chromatography, a peak of hemoglobins is satisfactorily obtained. In addition, a shoulder peak and a split peak are less likely to occur in all the obtained peaks. Thus, hemoglobins can be measured with high accuracy.

Hemolytic reagents including polyoxyethylene octyl phenyl ether having an average number of moles of an oxyethylene group added of 10 cause less carryover in measurement by cation exchange liquid chromatography. Thus, even when a large number of samples are continuously measured, hemoglobins can be measured with high accuracy.

Meanwhile, in Europe, the REACH regulation (Registration, Evaluation, Authorization and Restriction of Chemicals) has come into effect in 2007, and the use of specific chemicals have been limited. In 2017, products containing 0.1% by weight or more of polyoxyethylene octyl phenyl ether having an average number of moles of an oxyethylene group added of 10 have become subject to the REACH regulation. Also, in countries other than Europe, reduction of the amount of polyoxyethylene octyl phenyl ether having an average number of moles of an oxyethylene group added of 10 used contributes to the reduction of the environmental load and the improvement of the safety of a human body.

An object of the present invention is to provide a reagent for measuring hemoglobins capable of satisfactorily causing hemolysis and measuring hemoglobins with high accuracy. More specifically, an object of the present invention

is to provide a reagent for measuring hemoglobins capable of, without including polyoxyethylene octyl phenyl ether having an average number of moles of an oxyethylene group added of 10, causing hemolysis at a level equivalent to that of a reagent for measuring hemoglobins containing the component, and measuring hemoglobins with equivalent accuracy.

Another object of the present invention is to provide a method for measuring hemoglobins using the reagent for measuring hemoglobins.

**MEANS FOR SOLVING THE PROBLEMS**

According to a broad aspect of the present invention, there is provided a reagent for measuring hemoglobins used for measuring hemoglobins by cation exchange liquid chromatography, including: a nonionic surfactant or an amphoteric surfactant, the nonionic surfactant being Component A1 below, Component A2 below, Component A3 below, Component A4 below, Component A5 below, Component A6 below, Component A7 below, Component A8 below, or Component A9 below, and the amphoteric surfactant being Component B1 below, Component B2 below, or Component B3 below.

Component A1: Polyoxyethylene alkyl ether having an average number of moles of an oxyethylene group added of 8 or more and 20 or less, and a carbon number of an alkyl group of 12 or more and 17 or less
Component A2: Polyoxyethylene stearyl ether having an average number of moles of an oxyethylene group added of 40 or more and 60 or less
Component A3: Polyoxyethylene oleyl ether having an average number of moles of an oxyethylene group added of 9 or more and 15 or less
Component A4: Polyoxyethylene-sec-alkyl ether having an average number of moles of an oxyethylene group added of 6 or more and 10 or less, and a carbon number of an alkyl group of 11 or more and 15 or less
Component A5: Polyoxyethylene tridecyl ether having an average number of moles of an oxyethylene group added of 15
Component A6: Polyoxyethylene polyoxypropylene alkyl ether having an HLB value of 11 or more and 15 or less
Component A7: n-nonanoyl-N-methyl-D-glucamine
Component A8: n-octyl-β-D-glucopyranoside
Component A9: Saponin
Component B1: 3-[(3-cholamidopropyl)dimethylammonio]propane sulfonate
Component B2: 3-[(3-cholamidopropyl)dimethylammonio]-2-hydroxypropane sulfonate
Component B3: Lauryl dimethylamine oxide

In a particular aspect of the reagent for measuring hemoglobins according to the present invention, the reagent for measuring hemoglobins includes the nonionic surfactant, the nonionic surfactant includes the Component A1, the Component A2, the Component A3, the Component A4, the Component A5, or the Component A6, the Component A1 is polyoxyethylene lauryl ether having an average number of moles of an oxyethylene group added of 9, 12, or 19, or polyoxyethylene cetyl ether having an average number of moles of an oxyethylene group added of 13 or 20, the Component A2 is polyoxyethylene stearyl ether having an average number of moles of an oxyethylene group added of 50, the Component A3 is polyoxyethylene oleyl ether having an average number of moles of an oxyethylene group added of 9 or 13, the Component A4 is polyoxyethylene-sec-alkyl ether having an average number of moles of an oxyethylene group added of 7 or 9 and a carbon number of an alkyl group of 11 or more and 15 or less, and the Component A6 is polyoxyethylene polyoxypropylene alkyl ether having an HLB value of 12.5, 12.7, or 14.0.

In a particular aspect of the reagent for measuring hemoglobins according to the present invention, the reagent for measuring hemoglobins includes the nonionic surfactant, the nonionic surfactant includes the Component A1 or the Component A3, the Component A1 is polyoxyethylene alkyl ether having an average number of moles of an oxyethylene group added of 10 or more and 15 or less, and a carbon number of an alkyl group of 12 or more and 17 or less, and the Component A3 is polyoxyethylene oleyl ether having an average number of moles of an oxyethylene group added of 10 or more and 15 or less.

In a particular aspect of the reagent for measuring hemoglobins according to the present invention, the Component A1 is polyoxyethylene lauryl ether having an average number of moles of an oxyethylene group added of 12, or polyoxyethylene cetyl ether having an average number of moles of an oxyethylene group added of 13, and the Component A3 is polyoxyethylene oleyl ether having an average number of moles of an oxyethylene group added of 13.

In a particular aspect of the reagent for measuring hemoglobins according to the present invention, the reagent for measuring hemoglobins includes the nonionic surfactant, and the reagent for measuring hemoglobins has a content of the nonionic surfactant of 0.01% by weight or more and 1.0% by weight or less.

In a particular aspect of the reagent for measuring hemoglobins according to the present invention, the reagent

for measuring hemoglobins includes the amphoteric surfactant, and the reagent for measuring hemoglobins has a content of the amphoteric surfactant of 0.01% by weight or more and 1.0% by weight or less.

According to a broad aspect of the present invention, there is provided a method for measuring hemoglobins, including the steps of: mixing an erythrocyte-containing sample and the reagent for measuring hemoglobins to obtain a mixed solution; and measuring the mixed solution by cation exchange liquid chromatography.

## EFFECT OF THE INVENTION

The reagent for measuring hemoglobins according to the present invention is used for measuring hemoglobins by cation exchange liquid chromatography. The reagent for measuring hemoglobins according to the present invention includes a nonionic surfactant or an amphoteric surfactant. In the reagent for measuring hemoglobins according to the present invention, the nonionic surfactant is any of Components A1 to A9 below, or the amphoteric surfactant is any of Components B1 to B3 below. Component A1: Polyoxyethylene alkyl ether having an average number of moles of an oxyethylene group added of 8 or more and 20 or less, and a carbon number of an alkyl group of 12 or more and 17 or less. Component A2: Polyoxyethylene stearyl ether having an average number of moles of an oxyethylene group added of 40 or more and 60 or less. Component A3: Polyoxyethylene oleyl ether having an average number of moles of an oxyethylene group added of 9 or more and 15 or less. Component A4: Polyoxyethylene-sec-alkyl ether having an average number of moles of an oxyethylene group added of 6 or more and 10 or less, and a carbon number of an alkyl group of 11 or more and 15 or less. Component A5: Polyoxyethylene tridecyl ether having an average number of moles of an oxyethylene group added of 15. Component A6: Polyoxyethylene polyoxypropylene alkyl ether having an HLB value of 11 or more and 15 or less. Component A7: n-nonanoyl-N-methyl-D-glucamine. Component A8: n-octyl-β-D-glucopyranoside. Component A9: Saponin. Component B1: 3-[(3-cholamidopropyl)dimethylammonio]propane sulfonate. Component B2: 3-[(3-cholamidopropyl)dimethylammonio]-2-hydroxypropane sulfonate. Component B3: Lauryl dimethylamine oxide. The reagent for measuring hemoglobins according to the present invention has the above-mentioned configuration, and thus is capable of satisfactorily causing hemolysis and measuring hemoglobins with high accuracy.

## BRIEF DESCRIPTION OF DRAWINGS

[Fig. 1] Fig. 1 is an example of a chromatogram obtained when measurement is performed by cation exchange liquid chromatography using the reagent for measuring hemoglobins according to one embodiment of the present invention.

[Fig. 2] Fig. 2 is an example of a chromatogram obtained when measurement is performed by cation exchange liquid chromatography using a conventional reagent for measuring hemoglobins.

[Fig. 3] Figs. 3(a) and 3(b) are examples of chromatograms for evaluating the shape of the peak of hemoglobin A0.

[Fig. 4] Figs. 4(a) and 4(b) are examples of chromatograms for evaluating the peak shape of the first fraction.

[Fig. 5] Figs. 5(a) to 5(c) are examples of chromatograms for evaluating the height of the peak of hemoglobin A0.

[Fig. 6] Figs. 6 (a) and 6(b) are figures showing the relationship between the number of storage days and the value of hemoglobin A1c, and the relationship between the number of storage days and the variation in the value of hemoglobin A1c in a mixed solution of a reagent for measuring hemoglobins and whole blood.

[Fig. 7] Figs. 7(a) and 7(b) are figures showing the relationship between the number of storage days and the value of hemoglobin A1c, and the relationship between the number of storage days and the variation in the value of hemoglobin A1c in a mixed solution of a reagent for measuring hemoglobins and a hemoglobin A1c substance for measurement control (low concentration) (IRC-L).

[Fig. 8] Figs. 8(a) and 8(b) are figures showing the relationship between the number of storage days and the value of hemoglobin A1c, and the relationship between the number of storage days and the variation in the value of hemoglobin A1c in a mixed solution of a reagent for measuring hemoglobins and a hemoglobin A1c substance for measurement control (high concentration) (IRC-H).

[Fig. 9] Figs. 9(a) and 9(b) are figures showing column durability in a mixed solution of a reagent for measuring hemoglobins and a hemoglobin A1c substance for measurement control (high concentration) (IRC-H).

## MODES FOR CARRYING OUT THE INVENTION

Hereinafter, the present invention will be described in detail.

The reagent for measuring hemoglobins according to the present invention is used for measuring hemoglobins by cation exchange liquid chromatography. The reagent for measuring hemoglobins according to the present invention includes a nonionic surfactant or an amphoteric surfactant.

In the reagent for measuring hemoglobins according to the present invention, the nonionic surfactant is polyoxyethylene alkyl ether having an average number of moles of an oxyethylene group added of 8 or more and 20 or less, and a carbon number of an alkyl group of 12 or more and 17 or less, polyoxyethylene stearyl ether having an average number of moles of an oxyethylene group added of 40 or more and 60 or less, polyoxyethylene oleyl ether having an average number of moles of an oxyethylene group added of 9 or more and 15 or less, polyoxyethylene-sec-alkyl ether having an average number of moles of an oxyethylene group added of 6 or more and 10 or less, and a carbon number of an alkyl group of 11 or more and 15 or less, polyoxyethylene tridecyl ether having an average number of moles of an oxyethylene group added of 15, polyoxyethylene polyoxypropylene alkyl ether having an HLB value of 11 or more and 15 or less, n-nonanoyl-N-methyl-D-glucamine, n-octyl-β-D-glucopyranoside, or saponin.

In the reagent for measuring hemoglobins according to the present invention, the amphoteric surfactant is 3-[(3-cholamidopropyl)dimethylammonio]propane sulfonate, 3-[(3-cholamidopropyl)dimethylammonio]-2-hydroxypropane sulfonate, or lauryl dimethylamine oxide.

In the present specification, "Polyoxyethylene alkyl ether having an average number of moles of an oxyethylene group added of 8 or more and 20 or less, and a carbon number of an alkyl group of 12 or more and 17 or less" may be referred to as "Component A1".

In the present specification, for Component A1, "polyoxyethylene alkyl ether having an average number of moles of an oxyethylene group added of 10 or more and 15 or less, and a carbon number of an alkyl group of 12 or more and 17 or less" may be referred to as "Component A1'".

In the present specification, "Polyoxyethylene stearyl ether having an average number of moles of an oxyethylene group added of 40 or more and 60 or less" may be referred to as "Component A2".

In the present specification, "Polyoxyethylene oleyl ether having an average number of moles of an oxyethylene group added of 9 or more and 15 or less" may be referred to as "Component A3".

In the present specification, for Component A3, "Polyoxyethylene oleyl ether having an average number of moles of an oxyethylene group added of 10 or more and 15 or less" may be referred to as "Component A3'".

In the present specification, "Polyoxyethylene-sec-alkyl ether having an average number of moles of an oxyethylene group added of 6 or more and 10 or less, and a carbon number of an alkyl group of 11 or more and 15 or less" may be referred to as "Component A4".

In the present specification, "Polyoxyethylene tridecyl ether having an average number of moles of an oxyethylene group added of 15" may be referred to as "Component A5".

In the present specification, "Polyoxyethylene polyoxypropylene alkyl ether having an HLB value of 11 or more and 15 or less" may be referred to as "Component A6".

In the present specification, "n-nonanoyl-N-methyl-D-glucamine" may be referred to as "Component A7".

In the present specification, "n-octyl-β-D-glucopyranoside" may be referred to as "Component A8".

In the present specification, "Saponin" may be referred to as "Component A9".

In the present specification, "3-[(3-cholamidopropyl)dimethylammonio]propane sulfonate" may be referred to as "Component B1".

In the present specification, "3-[(3-cholamidopropyl)dimethylammonio]-2-hydroxypropane sulfonate" may be referred to as "Component B2".

In the present specification, "Lauryl dimethylamine oxide" may be referred to as "Component B3".

In the present specification, "Polyoxyethylene octyl phenyl ether having an average number of moles of an oxyethylene group added of 10" may be referred to as "Component X".

The reagent for measuring hemoglobins according to the present invention includes a nonionic surfactant or an amphoteric surfactant, and the nonionic surfactant is Component A1, Component A2, Component A3, Component A4, Component A5, Component A6, Component A7, Component A8, or Component A9, or the amphoteric surfactant is Component B1, Component B2, or Component B3.

The reagent for measuring hemoglobins according to the present invention has the above-mentioned configuration, and thus is capable of satisfactorily causing hemolysis and measuring hemoglobins with high accuracy.

A conventional reagent for measuring hemoglobins including Component X is capable of satisfactorily causing hemolysis and measuring hemoglobins with high accuracy. On the other hand, a reagent for measuring hemoglobins not including Component X is not capable of satisfactorily causing hemolysis and measuring hemoglobins with high accuracy.

In contrast, the reagent for measuring hemoglobins according to the present invention has the above-mentioned configuration, and thus is capable of, without including Component X, causing hemolysis at a level equivalent to that of a reagent for measuring hemoglobins including Component X, and measuring hemoglobins with equivalent accuracy.

The reagent for measuring hemoglobins according to the present invention is capable of satisfactorily causing hemolysis, and thus the peak of hemoglobins can be satisfactorily obtained when measurement is performed by cation exchange liquid chromatography. In addition, a shoulder peak and a split peak are less likely to occur in all the obtained peaks. Thus, hemoglobins can be measured with high accuracy. With the reagent for measuring hemoglobins according

to the present invention, carryover can be reduced. Thus, even when a large number of samples are continuously measured, hemoglobins can be measured with high accuracy.

At a test center, after a hemolytic reagent (a reagent for measuring hemoglobins) and a substance to be tested are mixed, the mixed solution may be stored for a certain period (for example, several days) before the mixed solution is measured by cation exchange chromatography. The reagent for measuring hemoglobins according to the present invention can increase the storage stability of a mixed solution obtained by mixing the reagent and the substance to be tested. The substance to be tested includes, in addition to an erythrocyte-containing sample such as blood (for example, blood of a subject), a hemoglobin-containing sample without an erythrocyte (for example, a standard substance for hemoglobins and a hemoglobin substance for measurement control). With the reagent for measuring hemoglobins according to the present invention, even when the mixed solution is stored for a certain period of time, the peak shape of hemoglobins is less likely to change before and after storage, and the measurement value is less likely to vary.

Particularly, when the reagent for measuring hemoglobins according to the present invention includes Component A1' or Component A3', the storage stability of the mixed solution obtained by mixing the reagent for measuring hemoglobins and the substance to be tested can be further increased, and the storage stability of the reagent for measuring hemoglobins can also be increased.

The reagent for measuring hemoglobins according to the present invention is used for measuring hemoglobins by cation exchange liquid chromatography. The reagent for measuring hemoglobins is preferably used for measuring hemoglobins by cation exchange high performance liquid chromatography.

Examples of the hemoglobins include normal hemoglobins such as hemoglobin A1a, hemoglobin A1b, hemoglobin F, hemoglobin A1c, hemoglobin A0, and hemoglobin A2, modified hemoglobins such as acetylated hemoglobin and carbamylated hemoglobin, and abnormal hemoglobins such as hemoglobin S and hemoglobin C.

The reagent for measuring hemoglobins according to the present invention is suitably used for measuring the value of the hemoglobin A1c (the concentration of hemoglobin A1c).

Fig. 1 is an example of a chromatogram obtained when measurement is performed by cation exchange liquid chromatography using the reagent for measuring hemoglobins according to one embodiment of the present invention.

Fig. 2 is an example of a chromatogram obtained when measurement is performed by cation exchange liquid chromatography using a conventional reagent for measuring hemoglobins (a conventional reagent for measuring hemoglobins including Component X).

In Figs. 1 and 2, the peak detected at about 19 seconds is the peak of hemoglobin A1c, and the peak detected at about 40 seconds is the peak of hemoglobin A0. Figs. 1 and 2 are, for example, examples of chromatograms when measurement is performed under the measurement conditions used in Examples below.

With the reagent for measuring hemoglobins according to the present invention, a peak shape equivalent to that of a conventional reagent for measuring hemoglobins including Component X can be obtained.

With the reagent for measuring hemoglobins according to the present invention, a component that is subject to the REACH regulation can be eliminated, and thus the influence on the environment can be reduced and the safety can be increased compared to the conventional reagent for measuring hemoglobins including Component X.

The reagent for measuring hemoglobins according to the present invention can include the nonionic surfactant, can include the amphoteric surfactant, or can include both of the nonionic surfactant and the amphoteric surfactant.

(Nonionic surfactant)

The reagent for measuring hemoglobins preferably includes a nonionic surfactant. The nonionic surfactant is any of Components A1 to A9. As the above-mentioned nonionic surfactant, only one of Components A1 to A9 can be used, or two or more can be used in combination.

<Component A1>

Component A1 is polyoxyethylene alkyl ether having an average number of moles of an oxyethylene group added of 8 or more and 20 or less, and a carbon number of an alkyl group of 12 or more and 17 or less. Component A1 is a component represented by the formula (1) below. As Component A1, only one type can be used, or two or more types can be used in combination.

$$R\text{-}O(CH_2CH_2O)_nH \ ... \qquad (1)$$

In the formula (1) above, R represents an alkyl group having 12 or more and 17 or less carbon atoms, and n represents a number of 8 or more and 20 or less.

In the case of polyoxyethylene alkyl ether having an average number of moles of an oxyethylene group added of less than 8 and a carbon number of an alkyl group of less than 12, the surfactant is less likely to dissolve and a reagent

for measuring hemoglobins may not be prepared. In the case of polyoxyethylene alkyl ether having an average number of moles of an oxyethylene group added of less than 8 and a carbon number of an alkyl group of less than 12, a good peak may not be obtained. In the case of polyoxyethylene alkyl ether having an average number of moles of an oxyethylene group added of more than 20 and a carbon number of an alkyl group of more than 17, a good peak may not be obtained or carryover may occur.

From the viewpoint of increasing the solubility of the surfactant, and from the viewpoint of satisfactorily causing hemolysis and measuring hemoglobins with high accuracy, the average number of moles of an oxyethylene group added in Component A1 is preferably 9 or more, more preferably 10 or more, further preferably 11 or more, and preferably 19 or less.

From the viewpoint of effectively exhibiting the effect of the present invention, and from the viewpoint of increasing the storage stability of the mixed solution obtained by mixing the reagent for measuring hemoglobins and the substance to be tested, the carbon number of the alkyl group of Component A1 is preferably 17 or less, more preferably 16 or less, and particularly preferably 12 or 16. When the carbon number of the alkyl group is 12, Component A1 is polyoxyethylene lauryl ether, and when the carbon number of the alkyl group is 16, Component A1 is polyoxyethylene cetyl ether.

Thus, Component A1 is particularly preferably polyoxyethylene lauryl ether having an average number of moles of an oxyethylene group added of 8 or more and 20 or less, or polyoxyethylene cetyl ether having an average number of moles of an oxyethylene group added of 8 or more and 20 or less.

When Component A1 is polyoxyethylene lauryl ether, the average number of moles of an oxyethylene group added in the polyoxyethylene lauryl ether is preferably 9 or more, more preferably 10 or more, further preferably 11 or more, and preferably 19 or less, more preferably 16 or less, further preferably 13 or less. The average number of moles of an oxyethylene group added in the polyoxyethylene lauryl ether is also preferably 9, 12, or 19, and most preferably 12. When the average number of moles of an oxyethylene group added is in the above-mentioned preferred range or value, the solubility of the surfactant can be further increased, the hemolysis can be satisfactorily performed, hemoglobins can be measured with even higher accuracy, and the storage stability of the mixed solution obtained by mixing the reagent for measuring hemoglobins and the substance to be tested can be further increased.

When Component A1 is polyoxyethylene cetyl ether, the average number of moles of an oxyethylene group added in the polyoxyethylene cetyl ether is preferably 10 or more, more preferably 12 or more, and preferably 19 or less, more preferably 16 or less, further preferably 14 or less. The average number of moles of an oxyethylene group added in the polyoxyethylene cetyl ether is also preferably 13 or 20, and most preferably 13. When the average number of moles of an oxyethylene group added is in the above-mentioned preferred range or value, the solubility of the surfactant can be further increased, the hemolysis can be satisfactorily performed, hemoglobins can be measured with even higher accuracy, and the storage stability of the mixed solution obtained by mixing the reagent for measuring hemoglobins and the substance to be tested can be further increased.

Thus, Component A1 is preferably polyoxyethylene lauryl ether having an average number of moles of an oxyethylene group added of 9, 12, or 19, or polyoxyethylene cetyl ether having an average number of moles of an oxyethylene group added of 13 or 20. Component A1 is most preferably polyoxyethylene lauryl ether having an average number of moles of an oxyethylene group added of 12, or polyoxyethylene cetyl ether having an average number of moles of an oxyethylene group added of 13.

From the viewpoint of effectively exhibiting the effect of the present invention, and from the viewpoint of increasing the storage stability of the reagent for measuring hemoglobins and the storage stability of the mixed solution obtained by mixing the reagent and the substance to be tested, Component A1 is preferably polyoxyethylene alkyl ether having an average number of moles of an oxyethylene group added of 10 or more and 15 or less, and a carbon number of an alkyl group of 12 or more and 17 or less. That is, Component A1 is preferably Component A1'.

The HLB (Hydrophilc Lipophilc Balance) value of Component A1 is preferably 10 or more, more preferably 12 or more, and preferably 18 or less, more preferably 16 or less. When the HLB value is greater than or equal to the lower limit, generation of micelles can be suppressed, and column clogging can be effectively suppressed. When the HLB value is less than or equal to the upper limit, the cell membrane of blood cell components can be sufficiently dissolved, and column clogging can be effectively suppressed.

The HLB value of Component A1 and the HLB value of each component described later are the HLB value determined by Griffin method. The HLB value is 0 or more and 20 or less. The smaller the HLB value, the stronger the hydrophobicity (lipophilicity), and the larger the HLB value, the stronger the hydrophilicity. The HLB value determined by Griffin method is calculated by the following formula.

$$\text{HLB value} = 20 \times (\text{molecular weight of hydrophilic group}/\text{molecular weight})$$

The clouding point of Component A1 is preferably 80°C or more, more preferably 90°C or more, and preferably 150°C or less, more preferably 120°C or less. When the clouding point is greater than or equal to the lower limit and less than or equal to the upper limit, the solubility of the surfactant can be increased, and the reagent for measuring hemoglobins can be sufficiently prepared.

The clouding point is the temperature at which a transparent or translucent liquid undergoes phase separation due to a temperature change, and as a result, the liquid becomes opaque. The clouding point is also generally referred to as the lower critical solution temperature.

The content of Component A1 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A1 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A1 is greater than or equal to the lower limit and less than or equal to the upper limit, the effect of the present invention can be even more effectively exhibited, and the storage stability of the mixed solution obtained by mixing the reagent for measuring hemoglobins and the substance to be tested can be further increased.

Examples of the commercially available product of Component A1 include "EMULGEN 109P" manufactured by Kao Corporation (polyoxyethylene lauryl ether having an average number of moles of an oxyethylene group added of 9, polyoxyethylene (9) lauryl ether), "EMULGEN 120" manufactured by Kao Corporation (polyoxyethylene lauryl ether having an average number of moles of an oxyethylene group added of 12, polyoxyethylene (12) lauryl ether), "EMULGEN 147" manufactured by Kao Corporation (polyoxyethylene lauryl ether having an average number of moles of an oxyethylene group added of 19, polyoxyethylene (19) lauryl ether), "EMULGEN 220" manufactured by Kao Corporation (polyoxyethylene cetyl ether having an average number of moles of an oxyethylene group added of 13, polyoxyethylene (13) cetyl ether), and "Brij (registered trademark) 58" manufactured by Sigma-Aldrich (polyoxyethylene cetyl ether having an average number of moles of an oxyethylene group added of 20, polyoxyethylene (20) cetyl ether).

<Component A2>

Component A2 is polyoxyethylene stearyl ether having an average number of moles of an oxyethylene group added of 40 or more and 60 or less. Component A2 is a component represented by the formula (2) below. As Component A2, only one type can be used, or two or more types can be used in combination.

$$C_{18}H_{37}O(CH_2CH_2O)_nH \ ... \qquad (2)$$

In the formula (2) above, n represents a number of 40 or more and 60 or less.

In the case of polyoxyethylene stearyl ether having an average number of moles of an oxyethylene group added of less than 40, the surfactant is less likely to dissolve and a reagent for measuring hemoglobins may not be prepared. In the case of polyoxyethylene stearyl ether having an average number of moles of an oxyethylene group added of less than 40, a good peak may not be obtained. In the case of polyoxyethylene stearyl ether having an average number of moles of an oxyethylene group added of more than 60, a good peak may not be obtained or carryover may occur.

From the viewpoint of increasing the solubility of the surfactant, and from the viewpoint of satisfactorily causing hemolysis and measuring hemoglobins with high accuracy, the average number of moles of an oxyethylene group added in Component A2 is preferably 45 or more, and preferably 55 or less. From the viewpoint of further increasing the solubility of the surfactant, and from the viewpoint of even more satisfactorily causing hemolysis and measuring hemoglobins with even higher accuracy, the average number of moles of an oxyethylene group added in Component A2 is most preferably 50.

The HLB value of Component A2 is preferably 13 or more, more preferably 15 or more, and preferably 19 or less, more preferably 18 or less. When the HLB value is greater than or equal to the lower limit, generation of micelles can be suppressed, and column clogging can be effectively suppressed. When the HLB value is less than or equal to the upper limit, the cell membrane of blood cell components can be sufficiently dissolved, and column clogging can be effectively suppressed.

The HLB value of Component A2 can be determined by the above-mentioned method.

The clouding point of Component A2 is preferably 80°C or more, more preferably 90°C or more. When the clouding point is greater than or equal to the lower limit, the solubility of the surfactant can be increased, and the reagent for measuring hemoglobins can be sufficiently prepared.

The content of Component A2 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A2 is preferably 1.0% by weight or less, more preferably 0.8% by weight or less, further preferably 0.6% by weight or less, particularly preferably 0.5% by weight or

less in 100% by weight of the reagent for measuring hemoglobins. When the content of Component A2 is greater than or equal to the lower limit and less than or equal to the upper limit, the effect of the present invention can be even more effectively exhibited.

Examples of the commercially available product of Component A2 include "EMULGEN 350" manufactured by Kao Corporation (polyoxyethylene stearyl ether having an average number of moles of an oxyethylene group added of 50, polyoxyethylene (50) stearyl ether).

<Component A3>

Component A3 is polyoxyethylene oleyl ether having an average number of moles of an oxyethylene group added of 9 or more and 15 or less. Component A3 is a component represented by the formula (3) below. As Component A3, only one type can be used, or two or more types can be used in combination.

$$C_{18}H_{35}O(CH_2CH_2O)_nH \ ... \qquad (3)$$

In the formula (3) above, n represents a number of 9 or more and 15 or less.

In the case of polyoxyethylene oleyl ether having an average number of moles of an oxyethylene group added of less than 9, the surfactant is less likely to dissolve and a reagent for measuring hemoglobins may not be prepared. In the case of polyoxyethylene oleyl ether having an average number of moles of an oxyethylene group added of less than 9, a good peak may not be obtained. In the case of polyoxyethylene oleyl ether having an average number of moles of an oxyethylene group added of more than 15, a good peak may not be obtained or carryover may occur.

From the viewpoint of increasing the solubility of the surfactant, and from the viewpoint of satisfactorily causing hemolysis and measuring hemoglobins with high accuracy, the average number of moles of an oxyethylene group added in Component A3 is preferably 14 or less. From the viewpoint of further increasing the solubility of the surfactant, and from the viewpoint of even more satisfactorily causing hemolysis and measuring hemoglobins with even higher accuracy, the average number of moles of an oxyethylene group added in Component A3 is particularly preferably 9 or 13, most preferably 13.

From the viewpoint of effectively exhibiting the effect of the present invention, and from the viewpoint of increasing the storage stability of the reagent for measuring hemoglobins and the storage stability of the mixed solution obtained by mixing the reagent and the substance to be tested, Component A3 is preferably polyoxyethylene oleyl ether having an average number of moles of an oxyethylene group added of 10 or more and 15 or less. That is, Component A3 is preferably Component A3'.

The HLB value of Component A3 is preferably 10 or more, more preferably 12 or more, and preferably 17 or less, more preferably 15 or less. When the HLB value is greater than or equal to the lower limit, generation of micelles can be suppressed, and column clogging can be effectively suppressed. When the HLB value is less than or equal to the upper limit, the cell membrane of blood cell components can be sufficiently dissolved, and column clogging can be effectively suppressed.

The HLB value of Component A3 can be determined by the above-mentioned method.

The clouding point of Component A3 is preferably 50°C or more, more preferably 85°C or more, and preferably 100°C or less, more preferably 95°C or less. When the clouding point is greater than or equal to the lower limit and less than or equal to the upper limit, the solubility of the surfactant can be increased, and the reagent for measuring hemoglobins can be sufficiently prepared.

The content of Component A3 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A3 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A3 is greater than or equal to the lower limit and less than or equal to the upper limit, the effect of the present invention can be even more effectively exhibited.

Examples of the commercially available product of Component A3 include "EMULGEN 409PV" manufactured by Kao Corporation (polyoxyethylene oleyl ether having an average number of moles of an oxyethylene group added of 9, polyoxyethylene (9) oleyl ether) and "EMULGEN 420" manufactured by Kao Corporation (polyoxyethylene oleyl ether having an average number of moles of an oxyethylene group added of 13, polyoxyethylene (13) oleyl ether).

<Component A4>

Component A4 is polyoxyethylene-sec-alkyl ether having an average number of moles of an oxyethylene group added of 6 or more and 10 or less, and a carbon number of an alkyl group of 11 or more and 15 or less. Component A4

is a component represented by the formula (4) below. Component A4 is an adduct of ethylene oxide and a secondary alcohol having 11 to 15 carbon atoms. Component A4 is different from Component A1. As Component A4, only one type can be used, or two or more types can be used in combination.

$$C_mH_{2m+1}O(CH_2CH_2O)_nH \text{ ...} \qquad (4)$$

In the formula (4) above, m represents a number of 11 or more and 15 or less, and n represents a number of 6 or more and 10 or less.

In the case of polyoxyethylene-sec-alkyl ether having an average number of moles of an oxyethylene group added of less than 6 and a carbon number of an alkyl group of 11 or more and 15 or less, the surfactant is less likely to dissolve and a reagent for measuring hemoglobins may not be prepared. In addition, in the case of polyoxyethylene-sec-alkyl ether having an average number of moles of an oxyethylene group added of less than 6 and a carbon number of an alkyl group of 11 or more and 15 or less, a good peak may not be obtained.

From the viewpoint of increasing the solubility of the surfactant, and from the viewpoint of satisfactorily causing hemolysis and measuring hemoglobins with high accuracy, the average number of moles of an oxyethylene group added in Component A4 is preferably 7 or more, preferably 9 or less, and more preferably 7 or 9.

The HLB value of Component A4 is preferably 10 or more, more preferably 12 or more, and preferably 17 or less, more preferably 15 or less. When the HLB value is greater than or equal to the lower limit, generation of micelles can be suppressed, and column clogging can be effectively suppressed. When the HLB value is less than or equal to the upper limit, the cell membrane of blood cell components can be sufficiently dissolved, and column clogging can be effectively suppressed.

The HLB value of Component A4 can be determined by the above-mentioned method.

The clouding point of Component A4 is preferably 30°C or more, and preferably 60°C or less. When the clouding point is greater than or equal to the lower limit and less than or equal to the upper limit, the solubility of the surfactant can be increased, and the reagent for measuring hemoglobins can be sufficiently prepared.

The content of Component A4 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A4 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A4 is greater than or equal to the lower limit and less than or equal to the upper limit, the effect of the present invention can be even more effectively exhibited.

Examples of the commercially available product of Component A4 include "EMULGEN 707" manufactured by Kao Corporation (polyoxyethylene-sec-alkyl ether having an average number of moles of an oxyethylene group added of 7 and a carbon number of an alkyl group of 11 or more and 15 or less) and "EMULGEN 709" manufactured by Kao Corporation (polyoxyethylene-sec-alkyl ether having an average number of moles of an oxyethylene group added of 9 and a carbon number of an alkyl group of 11 or more and 15 or less).

<Component A5>

Component A5 is polyoxyethylene tridecyl ether having an average number of moles of an oxyethylene group added of 15. Component A5 is a component represented by the formula (5) below. As Component A5, only one type can be used, or two or more types can be used in combination.

$$C_{13}H_{27}O(CH_2CH_2O)_{15}H \text{ ...} \qquad (5)$$

The content of Component A5 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A5 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A5 is greater than or equal to the lower limit and less than or equal to the upper limit, the effect of the present invention can be even more effectively exhibited.

Examples of the commercially available product of Component A5 include "LEOCOL TD-150" manufactured by Lion Corporation.

<Component A6>

Component A6 is polyoxyethylene polyoxypropylene alkyl ether having an HLB value of 11 or more and 15 or less. The polyoxyethylene polyoxypropylene alkyl ether has an oxypropylene group and an oxyethylene group. Component A6 is a component represented by the formula (6) below. As Component A6, only one type can be used, or two or more types can be used in combination.

$$R\text{-}(CH_2CH(CH_3)O)_m(CH_2CH_2O)_nH \ ... \qquad (6)$$

In the formula (6) above, R represents an alkyl group, m represents a number of 1 or more, and n represents a number of 1 or more.

The HLB value of Component A6 is preferably 11.5 or more, more preferably 12 or more, and preferably 14.5 or less. When the HLB value is greater than or equal to the lower limit, generation of micelles can be suppressed, and column clogging can be effectively suppressed. When the HLB value is less than or equal to the upper limit, the cell membrane of blood cell components can be sufficiently dissolved, and column clogging can be effectively suppressed. From the viewpoint of even more effectively exhibiting the effects of the present invention, and from the viewpoint of even more effectively suppressing the column clogging, the HLB value of Component A6 is preferably 12.5, 12.7, or 14.0.

The HLB value of Component A6 can be determined by the above-mentioned method.

The clouding point of Component A6 is preferably 30°C or more, more preferably 50°C or more, and preferably 95°C or less, more preferably 90°C or less. When the clouding point is greater than or equal to the lower limit and less than or equal to the upper limit, the solubility of the surfactant can be increased, and the reagent for measuring hemoglobins can be sufficiently prepared.

The content of Component A6 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A6 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A6 is greater than or equal to the lower limit and less than or equal to the upper limit, the effect of the present invention can be even more effectively exhibited.

Examples of the commercially available product of Component A6 include "EMULGEN LS-106" manufactured by Kao Corporation (polyoxyethylene polyoxypropylene alkyl ether having an HLB value of 12.5), "EMULGEN LS-114" manufactured by Kao Corporation (polyoxyethylene polyoxypropylene alkyl (C12 to 14) ether having an HLB value of 14.0), and "EMULGEN MS-110" manufactured by Kao Corporation (polyoxyethylene polyoxypropylene alkyl ether having an HLB value of 12.7).

<Component A7>

Component A7 is n-nonanoyl-N-methyl-D-glucamine.

The content of Component A7 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A7 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A7 is greater than or equal to the lower limit and less than or equal to the upper limit, the effect of the present invention can be even more effectively exhibited.

Examples of the commercially available product of Component A7 include "MEGA-9" manufactured by Dojindo Molecular Technologies, Inc.

<Component A8>

Component A8 is n-octyl-β-D-glucopyranoside.

The content of Component A8 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A8 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A8 is greater than or equal to the lower limit and less than or equal to the upper limit, the effect of the present invention can be even more effectively exhibited.

Examples of the commercially available product of Component A8 include "n-octyl-β-D-glucopyranoside" manufactured by Dojindo Molecular Technologies, Inc.

<Component A9>

Component A9 is saponin. As Component A9, only one type can be used, or two or more types can be used in combination.

Component A9 is preferably soybean saponin, more preferably soybean saponin represented by the formula (9) below.

[Chemical 1]

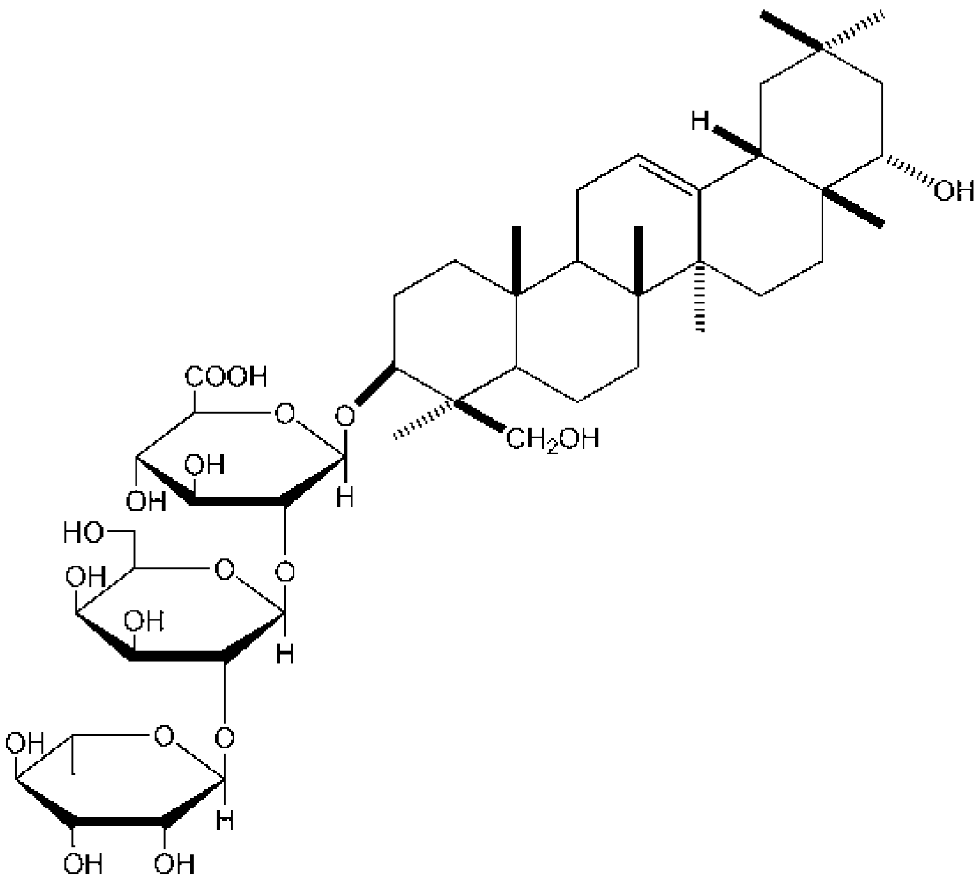

(9)

The content of Component A9 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component A9 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component A9 is greater than or equal to the lower limit and less than or equal to the upper limit, the effect of the present invention can be even more effectively exhibited.

Examples of the commercially available product of Component A9 include "Saponin" manufactured by NACALAI TESQUE, INC.

(Amphoteric surfactant)

The reagent for measuring hemoglobins preferably includes an amphoteric surfactant. The surfactant is any of Components B1 to B3. As the above-mentioned amphoteric surfactant, only one of Components B1 to B3 can be used, or two or more can be used in combination.

<Component B1>

Component B1 is 3-[(3-cholamidopropyl)dimethylammonio]propane sulfonate (CHAPS).

The content of Component B1 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component B1 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component B1 is greater than or equal to the lower limit and less than or equal to the upper limit, the effect of the present invention can be even more effectively exhibited.

Examples of the commercially available product of Component B1 include "CHAPS" manufactured by Dojindo Molecular Technologies, Inc.

<Component B2>

Component B2 is 3-[(3-cholamidopropyl)dimethylammonio]-2-hydroxypropane sulfonate (CHAPSO).

The content of Component B2 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component B2 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component B2 is greater than or equal to the lower limit and less than or equal to the upper limit, the effect of the present invention can be even more effectively exhibited.

Examples of the commercially available product of Component B2 include "CHAPSO" manufactured by Dojindo Molecular Technologies, Inc.

<Component B3>

Component B3 is lauryl dimethylamine oxide.

The content of Component B3 is preferably 0.01% by weight or more, more preferably 0.05% by weight or more, further preferably 0.08% by weight or more, particularly preferably 0.1% by weight or more in 100% by weight of the reagent for measuring hemoglobins. The content of Component B3 is preferably 1.0% by weight or less, more preferably 0.8% by weight or less, further preferably 0.6% by weight or less, particularly preferably 0.5% by weight or less in 100% by weight of the reagent for measuring hemoglobins. When the content of Component B3 is greater than or equal to the lower limit and less than or equal to the upper limit, the effect of the present invention can be even more effectively exhibited.

Examples of the commercially available product of Component B3 include "AMPHITOL 20N" manufactured by Kao Corporation.

(Buffering agent)

The reagent for measuring hemoglobins preferably includes a buffering agent. By inclusion of the buffering agent, variation in pH can be suppressed. As the buffering agent, only one type can be used, or two or more types can be used in combination.

Examples of the buffering agent include phosphates such as sodium phosphate, disodium hydrogen phosphate, sodium dihydrogen phosphate, potassium phosphate, dipotassium hydrogen phosphate, and potassium dihydrogen phosphate, carbonates such as sodium carbonate and sodium hydrogen carbonate, borate such as sodium borate, and carboxylic acid, dicarboxylic acid, carboxylic acid derivative, hydroxycarboxylic acid, aniline, aniline derivative, amino acid, an amine compound, an imidazole compound, an alcohol compound, ethylenediaminetetraacetic acid, pyrophosphoric acid, pyridine, cacodylic acid, glycerol phosphate, 2,4,6-collidine, N-ethylmorpholine, morpholine, 4-aminopyridine, ammonia, ephedrine, hydroxyproline, piperidine, tris(hydroxymethyl)aminomethane, and glycylglycine.

From the viewpoint of maintaining the pH of the reagent for measuring hemoglobins in a preferred range described below, the buffering agent is preferably phosphate.

The content of the buffering agent in the reagent for measuring hemoglobins is not particularly limited as long as buffer action is exhibited. The content of the buffering agent can be 0.01% by weight or more, can be 0.02% by weight or more, and can be 0.2% by weight or less, 0.1% by weight or less in 100% by weight of the reagent for measuring hemoglobins.

(Inorganic salt)

The reagent for measuring hemoglobins preferably includes an inorganic salt. By inclusion of the inorganic salt, the osmotic pressure can be satisfactorily adjusted. As the inorganic salt, only one type can be used, or two or more types can be used in combination.
Examples of the inorganic salt include sodium chloride, potassium chloride, sodium sulfate, and potassium sulfate.
From the viewpoint of maintaining the osmotic pressure of the reagent for measuring hemoglobins in the preferred range described below, the inorganic salt is preferably sodium chloride, potassium chloride, sodium sulfate, or potassium sulfate, and more preferably sodium chloride.
The content of the inorganic salt in the reagent for measuring hemoglobins is not particularly limited. The content of the inorganic salt can be 0.1% by weight or more, and can be 1.0% by weight or less in 100% by weight of the reagent for measuring hemoglobins.

(Water)

The reagent for measuring hemoglobins preferably includes water.
The content of water is preferably 80% by weight or more, more preferably 90% by weight or more, further preferably 95% by weight in 100% by weight of the reagent for measuring hemoglobins.

(Other components)

The reagent for measuring hemoglobins can include other components other than the above-mentioned components. Examples of the other components include a preservative, a hemoglobin stabilizer, and a pH adjuster. As the other components, only one type can be used, or two or more types can be used in combination.
Examples of the preservative include sodium azide, thymol, and sodium propionate.
Examples of the hemoglobin stabilizer include chelating agents such as ethylenediaminetetraacetic acid (EDTA), and glutathione.
Examples of the pH adjuster include acids such as hydrochloric acid, phosphoric acid, nitric acid, and sulfuric acid, and bases such as sodium hydroxide, potassium hydroxide, lithium hydroxide, magnesium hydroxide, barium hydroxide, and calcium hydroxide.

(Other details of reagent for measuring hemoglobins)

The reagent for measuring hemoglobins preferably includes the nonionic surfactant, more preferably includes, as the nonionic surfactant, Component A1, Component A2, Component A3, Component A4, Component A5, or Component A6, further preferably includes Component A1 or Component A3, and particularly preferably includes Component A1' or Component A3'. In this case, the effect of the present invention can be even more effectively exhibited. The storage stability of the mixed solution obtained by mixing the reagent for measuring hemoglobins and the substance to be tested can be further increased.
When the reagent for measuring hemoglobins includes the nonionic surfactant, the content of the nonionic surfactant (the total content of Components A1 to A9) is preferably 0.01% by weight or more, more preferably 0.05% by weight or more, further preferably 0.08% by weight or more, and particularly preferably 0.1% by weight or more in 100% by weight of the reagent for measuring hemoglobins. The content of the nonionic surfactant (the total content of Components A1 to A9) is preferably 1.0% by weight or less, more preferably 0.8% by weight or less, further preferably 0.6% by weight or less, particularly preferably 0.5% by weight or less in 100% by weight of the reagent for measuring hemoglobins. When the content of the nonionic surfactant (the total content of Components A1 to A9) is greater than or equal to the lower limit and less than or equal to the upper limit, the effect of the present invention can be even more effectively exhibited. The storage stability of the mixed solution obtained by mixing the reagent for measuring hemoglobins and the substance to be tested can be further increased.
When the reagent for measuring hemoglobins includes the amphoteric surfactant, the content of the amphoteric surfactant (the total content of Components B1 to B3) is preferably 0.01% by weight or more, more preferably 0.05% by weight or more, further preferably 0.08% by weight or more, and particularly preferably 0.1% by weight or more in 100% by weight of the reagent for measuring hemoglobins. The content of the amphoteric surfactant (the total content of Components B1 to B3) is preferably 1.0% by weight or less, more preferably 0.8% by weight or less, further preferably 0.6% by weight or less, particularly preferably 0.5% by weight or less in 100% by weight of the reagent for measuring hemoglobins. When the content of the amphoteric surfactant (the total content of Components B1 to B3) is greater than or equal to the lower limit and less than or equal to the upper limit, the effect of the present invention can be even more

effectively exhibited.

The reagent for measuring hemoglobins preferably does not include Component X. In the present invention, the effects of the present invention can be exhibited without including Component X. In the present invention, the storage stability of the mixed solution obtained by mixing the reagent for measuring hemoglobins and the substance to be tested can be increased without including Component X. However, the reagent for measuring hemoglobins can include Component X. For example, the reagent for measuring hemoglobins can include Component X at a concentration not subject to the REACH regulation (the content of Component X of less than 0.1% by weight in 100% by weight of the reagent for measuring hemoglobins).

The pH of the reagent for measuring hemoglobins is preferably 6.0 or more, more preferably 7.0 or more, and preferably 8.5 or less, more preferably 8.0 or less. When the pH is greater than or equal to the lower limit and less than or equal to the upper limit, hemolysis can be even more satisfactorily caused.

The osmotic pressure of the reagent for measuring hemoglobins is preferably 50 mOsm or more, more preferably 75 mOsm or more, and preferably 200 mOsm or less, more preferably 150 mOsm or less. When the osmotic pressure is greater than or equal to the lower limit and less than or equal to the upper limit, hemolysis can be even more satisfactorily caused.

The osmotic pressure can be measured using an osmometer (for example, "Osmometer 3250" manufactured by Advanced Instruments, Inc.).

The reagent for measuring hemoglobins is suitably used for hemolyzing erythrocyte. The reagent for measuring hemoglobins is preferably a hemolytic reagent.

The reagent for measuring hemoglobins is suitably mixed with a substance to be tested and used. Examples of the substance to be tested include an erythrocyte-containing sample such as blood, a standard substance for hemoglobins, and a hemoglobin substance for measurement control.

The reagent for measuring hemoglobins is suitably used as a diluting solution for dissolving or diluting a sample not containing erythrocyte and having a known hemoglobin concentration (a standard substance for hemoglobins, a hemoglobin substance for measurement control and the like). Further, the reagent for measuring hemoglobins is also suitably used as a washing liquid for a liquid chromatograph and a washing liquid for a column.

(Method for measuring hemoglobins)

The method for measuring hemoglobins according to the present invention includes the steps of: mixing an erythrocyte-containing sample and the reagent for measuring hemoglobins to obtain a mixed solution; and measuring the mixed solution by cation exchange liquid chromatography.

The cation exchange liquid chromatography is preferably cation exchange high performance liquid chromatography.

Examples of the erythrocyte-containing sample include blood.

Generally, in the cation exchange liquid chromatography, in addition to a mixed solution prepared from blood collected from a patient or the like (an erythrocyte-containing sample), a mixed solution prepared from a sample not containing erythrocyte and having a known concentration of hemoglobins to be measured is also measured. Examples of the sample not containing erythrocyte and having a known concentration of hemoglobins to be measured include a standard substance for hemoglobins to be measured (hemoglobins to be measured having a known concentration), and a hemoglobin substance for measurement control to be measured (hemoglobins to be measured having a known concentration; multiple concentrations such as a low concentration, a medium concentration, and a high concentration are preferably measured). The standard substance for hemoglobins and the hemoglobin substance for measurement control are preferably a sample prepared at time of use, a freeze-dried sample, or a frozen sample. The standard substance for hemoglobins and the hemoglobin substance for measurement control are commercially available as a freeze-dried product or a frozen product.

In the method for measuring hemoglobins, the concentration of hemoglobins is preferably determined, for example, as follows. (1) The erythrocyte-containing sample and the reagent for measuring hemoglobins are mixed to obtain a mixed solution. (2) The mixed solution is measured by cation exchange liquid chromatography. (3) The ratio of the peak area of hemoglobins to be measured to the total of the peak areas of hemoglobins (the peak area of hemoglobins to be measured/the total of the peak areas of hemoglobins) is taken as the hemoglobin value (the concentration of hemoglobins) to be measured. For example, when the value of hemoglobin A1c is determined, in the above (3), the ratio of the peak area of hemoglobin A1c to the total of the peak areas of hemoglobins (the peak area of hemoglobin A1c/the total of the peak areas of hemoglobins) is taken as the value of hemoglobin A1c.

In the method for measuring hemoglobins, the concentration of hemoglobins can also be determined, for example, as follows. (1) The erythrocyte-containing sample and the reagent for measuring hemoglobins are mixed to obtain a first mixed solution. (2) A hemoglobin-containing sample having a known concentration of hemoglobins to be measured and the reagent for measuring hemoglobins are mixed to obtain a second mixed solution. (3) The first mixed

solution and the second mixed solution are measured by cation exchange liquid chromatography. (4) The measurement value obtained by measuring the mixed solution containing the hemoglobin-containing sample having a known concentration of hemoglobins to be measured by cation exchange liquid chromatography, and the measurement value obtained by measuring the mixed solution containing the erythrocyte-containing sample are compared to determine the concentration of hemoglobins in the erythrocyte-containing sample.

From the viewpoint of even more satisfactorily causing hemolysis, the reagent for measuring hemoglobins is preferably mixed in an amount of preferably 25 mL or more, more preferably 50 mL or more, and preferably 400 mL or less, more preferably 200 mL or less relative to 1 mL of the erythrocyte-containing sample.

In the cation exchange liquid chromatography, measurement is preferably performed by a salt concentration gradient or a pH gradient. The gradient can be a linear gradient or a step gradient.

Two or more eluents are preferably used in the gradient measurement. When measurement is performed by the salt concentration gradient, as the eluent, an eluent having a low salt concentration and an eluent having a high salt concentration (for example, an eluent having an NaCl concentration of 50 mM and an eluent having an NaCl concentration of 200 mM) can be used. When measurement is performed by the pH gradient, two types of eluents having different pHs (for example, an eluent at pH 5.4 and an eluent at pH 8.0) can be used. A commercially available product can also be used as the eluent. Examples of the commercially available product of the eluent include "Eluent 80A", "Eluent 80B", "Eluent 60A-VP/TP", "Eluent 60B-VP/TP", and "Eluent 60C-VP" manufactured by ARKRAY, Inc.

As the cation exchange column used in the cation exchange liquid chromatography, a conventionally known cation exchange column can be used. The cation exchange column is preferably a column packed with a packing material having a cation exchange group such as a carboxyl group, a sulfonic acid group, or a phosphoric acid group. Examples of the commercially available product of the cation exchange column include "COLUMN UNIT 80" and "COLUMN UNIT HSVI-VP" manufactured by ARKRAY, Inc.

Hereinafter, the present invention will be specifically described by giving Examples, Reference Example, and Comparative Examples. The present invention is not limited to the following Examples.

(Examples 1 to 30, Reference Example A, and Comparative Examples 1 to 69)

Preparation of reagent for measuring hemoglobins:

Potassium dihydrogen phosphate, dipotassium hydrogen phosphate, sodium chloride, sodium azide, and water were mixed to obtain a first composition. Each surfactant shown in Tables 2 to 6 was added to the obtained first composition and the mixture was mixed to prepare a reagent for measuring hemoglobins. The composition of the obtained reagent for measuring hemoglobins is shown in Table 1 below. The type, the content, the HLB value, and the clouding point of the surfactant in Table 1 are shown in Tables 2 to 6. The HLB value of the surfactant is the value measured by the above-mentioned method.

[Table 1]

| | | Content in 100% by weight of reagent for measuring hemoglobins |
|---|---|---|
| Potassium dihydrogen phosphate | % by weight | 0.013 |
| Dipotassium hydrogen phosphate | % by weight | 0.052 |
| Sodium chloride | % by weight | 0.233 |
| Sodium azide | % by weight | 0.02 |
| Surfactant * | % by weight | * |
| Water | % by weight | Remainder |
| Total | % by weight | 100 |
| * Type and content of surfactants are shown in Tables 2 to 6. | | |

(Evaluation)

(1) Solubility of surfactant

The solubility of the surfactant when the surfactant was added to the first composition and the mixture was

mixed was visually checked. The solubility of the surfactant was judged based on the following criteria.

[Criteria for judging solubility of surfactant]

○○: Surfactant is completely dissolved in less than 15 minutes

○: Surfactant is completely dissolved in 15 minutes or more and less than 30 minutes

Δ: Surfactant is completely dissolved in 30 minutes or more and less than 1 hour

× : Surfactant is completely dissolved in 1 hour or more, or is not dissolved

(2) Measurement by cation exchange liquid chromatography

Whole blood (erythrocyte-containing sample) contained in a blood collection tube (manufactured by SEKISUI MEDICAL CO., LTD.) was prepared. The obtained reagent for measuring hemoglobins (100 mL) was mixed with whole blood (1 mL) to obtain a mixed solution. The obtained mixed solution was measured under the following conditions. The reagent for measuring hemoglobins that had the judgement result of × in "(1) Solubility of surfactant" was not measured by cation exchange liquid chromatography.

Cation exchange chromatography conditions:

HPLC instrument: glycohemoglobin analyzer ("HA-8180" manufactured by ARKRAY, Inc.)
Cation exchange column: COLUMN UNIT 80 (manufactured by ARKRAY, Inc.)
Eluent A: ELUENT 80A (manufactured by ARKRAY, Inc.)
Eluent B: ELUENT 80B (manufactured by ARKRAY, Inc.)

The measurement was performed by the measurement method installed in the HPLC instrument.

(2-1) Peak separation pattern

The following i) to iii) were checked for the chromatogram obtained by measuring the mixed solution. In Reference Example A, a peak pattern of a chromatogram shown in Fig. 2 was obtained. In the obtained chromatogram, the peak detected at about 19 seconds is the peak of hemoglobin A1c, and the peak detected at about 40 seconds is the peak of hemoglobin A0.

i) Separation of peak of hemoglobin A1c

The peak height of hemoglobin A1c obtained in Reference Example A was compared with the peak height of hemoglobin A1c obtained in Examples and Comparative Examples. When the obtained peak height of hemoglobin A1c was decreased 50 or more based on absorbance compared with the peak height of hemoglobin A1c obtained in Reference Example A, the separation of the peak of hemoglobin A1c was judged to be good, and when it was decreased more than 50, it was judged to be bad.

ii) Shape of peak of hemoglobin A0

In Reference Example A, no peak was detected in the peak tail part of the peak of hemoglobin A0. The shape of the peak tail of the peak of hemoglobin A0 obtained in Examples and Comparative Examples was checked. In this peak tail part, when no peak was detected, the shape of the peak of hemoglobin A0 was judged to be good, and when a peak was detected, it was judged to be bad.

An example of a chromatogram judged to be good is shown in Fig. 3(a), and an example of a chromatogram judged to be bad is shown in Fig. 3 (b) .

iii) Peak shape of first fraction

In Reference Example A, no shoulder peak was detected in the peak of the first fraction. The peak shape of the fast fraction obtained in Examples and Comparative Examples was checked. In the peak of the first fraction, when

no shoulder peak was detected, the peak shape of the first fraction was judged to be good, and when a shoulder peak was detected, it was judged to be bad.
An example of a chromatogram judged to be good is shown in Fig. 4(a), and an example of a chromatogram judged to be bad is shown in Fig. 4(b). The peak indicated by the arrow in Fig. 4(b) is the shoulder peak.

[Criteria for judging peak separation pattern]

○: All judgments in i) to iii) are good
×: Any of judgments in i) to iii) is bad

(2-2) Carryover

Following the measurement of the mixed solution obtained by mixing whole blood and the reagent for measuring hemoglobins, only the reagent for measuring hemoglobins was continuously measured 5 times. The following i) to iii) were checked for the obtained chromatogram.

i) Height of peak of hemoglobin A0

In the first measurement of the reagent for measuring hemoglobins, the peak height of hemoglobin A0 obtained in Reference Example A was compared with the peak height of hemoglobin A0 obtained in Examples and Comparative Examples. When the obtained peak height of hemoglobin A0 was not increased 100 or more based on absorbance compared with the peak height of hemoglobin A0 obtained in Reference Example A, the judgement of good was given, and when it was increased more than 100, the judgement of bad was given.
An example of the chromatogram obtained in Reference Example A is shown in Fig. 5(a), an example of a chromatogram judged to be good is shown in Fig. 5(b), and an example of a chromatogram judged to be bad is shown in Fig. 5(c).

ii) Presence or absence of peak of hemoglobin A1c

In the first measurement of the reagent for measuring hemoglobins, the presence or absence of the peak of hemoglobin A1c was checked. When no peak of hemoglobin A1c was observed, the judgement of good was given, and when the peak of hemoglobin A1c was observed, the judgement of bad given.

iii) Presence or absence of drift occurrence

In the first measurement of the reagent for measuring hemoglobins, the presence or absence of the drift of the peak of hemoglobin A0 was checked. When no drift was observed, the judgement of good was given, and when the drift was observed, the judgement of bad was given.

[Criteria for judging carryover]

○: All judgments in i) to iii) are good
×: Any of judgments in i) to iii) is bad

(3) pH

The pH of the reagent for measuring hemoglobins obtained in Reference Example A and Examples was measured using a pH meter ("F-52" manufactured by HORIBA, Ltd.).

(4) Osmotic pressure

The osmotic pressure of the reagent for measuring hemoglobins obtained in Reference Example A and Examples was measured using an osmometer ("Osmometer 3250" manufactured by Advanced Instruments, Inc.).
The compositions and results are shown in Tables 2 to 6 below. In Tables 2, 3, and 5, the value represented

by "E.O." means the average number of moles of an oxyethylene group added.

[Table 2]

| | | | Product name | Manufacturer | Name of component | Clouding point (°C) | HLB value | Content | Evaluation | | | | |
|---|---|---|---|---|---|---|---|---|---|---|---|---|---|
| | | | | | | | | | Solubility | Peak separation pattern | Carryover | pH | Osmotic pressure (mOsm) |
| Reference Example A | Nonionic surfactant | Component X | Triton X-100 | NACALAI TESQUE, INC. | Polyoxyethylene octyl phenyl ether (10 E.O.) | 66 | 13.5 | 0.1% | ○ | ○ | ○ | 7.50 | 100 |
| Comparative Example 1 | | | Brij35 | Sigma-Aldrich | Polyoxyethylene lauryl ether (23 E.O.) | - | - | 0.5% | △ | × | × | - | - |
| Comparative Example 2 | | | EMULGEN 103 | Kao Corporation | Polyoxyethylene lauryl ether (3E.O.) | - | 8.1 | 0.5% | × | - | - | - | - |
| Comparative Example 3 | | | EMULGEN 104P | Kao Corporation | Polyoxyethylene lauryl ether (4E.O.) | - | 9.6 | 0.5% | × | - | - | - | - |
| Comparative Example 4 | | | EMULGEN 105 | Kao Corporation | Polyoxyethylene lauryl ether (4E.O.) | - | 9.7 | 0.5% | × | - | - | - | - |
| Comparative Example 5 | | | EMULGEN 106 | Kao Corporation | Polyoxyethylene lauryl ether (5E.O.) | - | 10.5 | 0.5% | × | - | - | - | - |
| Comparative Example 6 | | | EMULGEN 108 | Kao Corporation | Polyoxyethylene lauryl ether (6E.O.) | 40 | 12.1 | 0.5% | ○ | × | × | - | - |
| Example 1 | | Component A1 | EMULGEN 109P | Kao Corporation | Polyoxyethylene lauryl ether (9E.O.) | 83 | 13.6 | 0.5% | ○○ | ○ | ○ | 7.26 | 101 |
| Example 2 | | Component A1 | EMULGEN 120 | Kao Corporation | Polyoxyethylene lauryl ether (12E.O.) | 98 | 15.3 | 0.5% | ○ | ○ | ○ | 7.35 | 103 |
| Example 3 | | Component A1 | EMULGEN 120 | Kao Corporation | Polyoxyethylene lauryl ether (12E.O.) | 98 | 15.3 | 0.1% | ○ | ○ | ○ | 7.35 | 103 |
| Example 4 | | Component A1 | EMULGEN 147 | Kao Corporation | Polyoxyethylene lauryl ether (19E.O.) | >100 | 16.3 | 0.5% | ○ | ○ | ○ | 7.32 | 104 |
| Comparative Example 7 | | | EMULGEN 123P | Kao Corporation | Polyoxyethylene lauryl ether (23 E.O.) | >100 | 16.9 | 0.5% | ○ | × | × | - | - |
| Comparative Example 8 | | | EMULGEN 130K | Kao Corporation | Polyoxyethylene lauryl ether (41E.O.) | >100 | 18.1 | 0.5% | ○ | × | × | - | - |
| Comparative Example 9 | | | EMULGEN 150 | Kao Corporation | Polyoxyethylene lauryl ether (47E.O.) | >100 | 18.4 | 0.5% | ○ | × | × | - | - |
| Comparative Example 10 | | | EMULGEN 210P | Kao Corporation | Polyoxyethylene cetyl ether (7E.O.) | - | 10.7 | 0.5% | × | - | - | - | - |
| Example 5 | | Component A1 | EMULGEN 220 | Kao Corporation | Polyoxyethylene cetyl ether (13E.O.) | 98 | 14.2 | 0.5% | △ | ○ | ○ | 7.36 | 100 |
| Example 6 | | Component A1 | EMULGEN 220 | Kao Corporation | Polyoxyethylene cetyl ether (13E.O.) | 98 | 14.2 | 0.1% | △ | ○ | ○ | 7.36 | 100 |
| Example 7 | | Component A1 | Brij58 | Sigma-Aldrich | Polyoxyethylene cetyl ether (20E.O.) | - | - | 0.5% | △ | ○ | ○ | 7.34 | 101 |
| Comparative Example 11 | | | EMULGEN 306P | Kao Corporation | Polyoxyethylene stearyl ether (6E.O.) | - | 9.4 | 0.5% | × | - | - | - | - |
| Comparative Example 12 | | | EMULGEN 320P | Kao Corporation | Polyoxyethylene stearyl ether (12E.O.) | 91 | 13.9 | 0.5% | × | - | - | - | - |
| Example 8 | | Component A2 | EMULGEN 350 | Kao Corporation | Polyoxyethylene stearyl ether (50E.O.) | >100 | 17.8 | 0.5% | △ | ○ | ○ | 7.41 | 101 |

[Table 3]

| | | | Product name | Manufacturer | Name of component | Clouding point (°C) | HLB value | Content | Evaluation | | | | |
|---|---|---|---|---|---|---|---|---|---|---|---|---|---|
| | | | | | | | | | Solubility | Peak separation pattern | Carryover | pH | Osmotic pressure (mOsm) |
| Comparative Example 13 | Nonionic surfactant | | EMULGEN 404 | Kao Corporation | Polyoxyethylene oleyl ether (4E.O.) | - | 8.8 | 0.5% | × | - | - | - | - |
| Comparative Example 14 | | | EMULGEN 408 | Kao Corporation | Polyoxyethylene oleyl ether (8E.O.) | - | 10 | 0.5% | × | - | - | - | - |
| Example 9 | | Component A3 | EMULGEN 409PV | Kao Corporation | Polyoxyethylene oleyl ether (9E.O.) | 55 | 12 | 0.5% | △ | ○ | ○ | 7.39 | 100 |
| Example 10 | | Component A3 | EMULGEN 420 | Kao Corporation | Polyoxyethylene oleyl ether (13E.O.) | 91 | 13.6 | 0.5% | ○○ | ○ | ○ | 7.38 | 101 |
| Example 11 | | Component A3 | EMULGEN 420 | Kao Corporation | Polyoxyethylene oleyl ether (13E.O.) | 91 | 13.6 | 0.1% | ○○ | ○ | ○ | 7.38 | 101 |
| Comparative Example 15 | | | EMULGEN 430 | Kao Corporation | Polyoxyethylene oleyl ether (30E.O.) | >100 | 16.2 | 0.5% | △ | × | × | - | - |
| Comparative Example 16 | | | EMULGEN 705 | Kao Corporation | Polyoxyethylene-sec-alkyl ether (carbon number of an alkyl group of 11 or more and 15 or less, 5E.O.) | - | 10.5 | 0.5% | × | - | - | - | - |
| Example 12 | | Component A4 | EMULGEN 707 | Kao Corporation | Polyoxyethylene-sec-alkyl ether (carbon number of an alkyl group of 11 or more and 15 or less, 7E.O.) | 33 | 12.1 | 0.5% | ○○ | ○ | ○ | 7.31 | 104 |
| Example 13 | | Component A4 | EMULGEN 707 | Kao Corporation | Polyoxyethylene-sec-alkyl ether (carbon number of an alkyl group of 11 or more and 15 or less, 7E.O.) | 33 | 12.1 | 0.1% | ○○ | ○ | ○ | 7.31 | 104 |
| Example 14 | | Component A4 | EMULGEN 709 | Kao Corporation | Polyoxyethylene-sec-alkyl ether (carbon number of an alkyl group of 11 or more and 15 or less, 9E.O.) | 56 | 13.3 | 0.5% | ○○ | ○ | ○ | 7.22 | 103 |
| Example 15 | | Component A4 | EMULGEN 709 | Kao Corporation | Polyoxyethylene-sec-alkyl ether (carbon number of an alkyl group of 11 or more and 15 or less, 9E.O.) | 56 | 13.3 | 0.1% | ○○ | ○ | ○ | 7.22 | 103 |
| Comparative Example 17 | | | EMULGEN 1108 | Kao Corporation | Polyoxyethylene alkyl ether (carbon number of an alkyl group of 11, 8 E.O.) | 66 | 13.5 | 0.5% | ○ | × | × | - | - |
| Comparative Example 18 | | | EMULGEN 4085 | Kao Corporation | Polyoxyethylene myristyl ether (80E.O.) | >100 | 18.9 | 0.5% | ○ | × | × | - | - |
| Comparative Example 19 | | | EMULGEN 2020G-HA | Kao Corporation | Polyoxyethylene octyldodecyl ether (20 E.O.) | - | 13 | 0.5% | ○ | × | × | - | - |
| Comparative Example 20 | | | EMULGEN 2025G | Kao Corporation | Polyoxyethylene octyldodecyl ether (25 E.O.) | - | 15.7 | 0.5% | ○ | × | × | - | - |
| Example 16 | | Component A5 | LEOCOL TD-150 | Lion Corporation | Polyoxyethylene tridecyl ether (15 E.O.) | 75 | 15.4 | 0.5% | ○ | ○ | ○ | 7.33 | 103 |
| Example 17 | | Component A5 | LEOCOL TD-150 | Lion Corporation | Polyoxyethylene tridecyl ether (15 E.O.) | 75 | 15.4 | 0.1% | ○ | ○ | ○ | 7.33 | 103 |

[Table 4]

[Table 5]

| | | | Product name | Manufacturer | Name of component | Clouding point (°C) | HLB value | Content | Evaluation: Solubility | Evaluation: Peak separation pattern | Evaluation: Carryover | Evaluation: pH | Evaluation: Osmotic pressure (mOsm) |
|---|---|---|---|---|---|---|---|---|---|---|---|---|---|
| Comparative Example 21 | Nonionic surfactant | | EMULGEN A-60 | Kao Corporation | Polyoxyethylene distyrenated phenyl ether | - | - | 0.5% | ○ | × | × | - | - |
| Comparative Example 22 | | | EMULGEN A-90 | Kao Corporation | Polyoxyethylene distyrenated phenyl ether | - | - | 0.5% | ○ | × | × | - | - |
| Comparative Example 23 | | | EMULGEN B-66 | Kao Corporation | Polyoxyethylene tribenzyl phenyl ether | - | - | 0.5% | × | - | - | - | - |
| Comparative Example 24 | | | TWEEN20 | Tokyo Chemical Industry Co., Ltd. | Polyoxyethylene sorbitan monolaurate | - | - | 0.5% | ○ | × | × | - | - |
| Comparative Example 25 | | | TWEEN40 | Tokyo Chemical Industry Co., Ltd. | Polyoxyethylene sorbitan monopalmitate | - | - | 0.5% | × | - | - | - | - |
| Comparative Example 26 | | | TWEEN60 | Tokyo Chemical Industry Co., Ltd. | Polyoxyethylene sorbitan monostearate | - | - | 0.5% | × | - | - | - | - |
| Comparative Example 27 | | | TWEEN80 | Tokyo Chemical Industry Co., Ltd. | Polyoxyethylene sorbitan monooleate | - | - | 0.5% | ○ | × | × | - | - |
| Comparative Example 28 | | | TWEEN85 | Tokyo Chemical Industry Co., Ltd. | Polyoxyethylene sorbitan trioleate | - | - | 0.5% | ○ | × | × | - | - |
| Comparative Example 29 | | | RHEODOL TW-IS399C | Kao Corporation | Polyoxyethylene sorbitan triisostearate | - | - | 0.5% | ○ | × | × | - | - |
| Comparative Example 30 | | | RHEODOL SP-L10 | Kao Corporation | Sorbitan monolaurate | - | - | 0.5% | × | - | - | - | - |
| Comparative Example 31 | | | RHEODOL SP-P10 | Kao Corporation | Sorbitan monopalmitate | - | - | 0.5% | × | - | - | - | - |
| Comparative Example 32 | | | RHEODOL SP-S10V | Kao Corporation | Sorbitan monostearate | - | - | 0.5% | × | - | - | - | - |
| Comparative Example 33 | | | RHEODOL SP-S30V | Kao Corporation | Sorbitan tristearate | - | - | 0.5% | × | - | - | - | - |
| Comparative Example 34 | | | RHEODOL SP-O10V | Kao Corporation | Sorbitan monooleate | - | - | 0.5% | × | - | - | - | - |
| Comparative Example 35 | | | RHEODOL SP-O30V | Kao Corporation | Sorbitan trioleate | - | - | 0.5% | × | - | - | - | - |
| Comparative Example 36 | | | RHEODOL AO-15V | Kao Corporation | Sorbitan sesquioleate | - | - | 0.5% | × | - | - | - | - |
| Comparative Example 37 | | | EMANON 1112 | Kao Corporation | Polyethylene glycol monolaurate | - | 13.7 | 0.5% | ○ | × | ○ | - | - |
| Comparative Example 38 | | | EMANON 3199V | Kao Corporation | Polyethylene glycol monostearate | - | 19.4 | 0.5% | × | - | - | - | - |
| Comparative Example 39 | | | EMANON 3299VB | Kao Corporation | Polyethylene glycol distearate | - | 18.9 | 0.5% | × | - | - | - | - |
| Comparative Example 40 | | | EMANON 3299RV | Kao Corporation | Polyethylene glycol distearate | - | 19.2 | 0.5% | × | - | - | - | - |
| Comparative Example 41 | | | AMINON PK-02S | Kao Corporation | Alkyl alkanolamide | - | - | 0.5% | × | - | - | - | - |
| Comparative Example 42 | | | AMIET 105 | Kao Corporation | Polyoxyethylene alkylamine | - | - | 0.5% | ○ | × | × | - | - |
| Comparative Example 43 | | | AMIET 320 | Kao Corporation | Polyoxyethylene alkylamine | - | - | 0.5% | ○ | × | × | - | - |

[Table 6]

| | | | Product name | Manufacturer | Name of component | Clouding point (°C) | HLB value | Content | Evaluation | | | | |
|---|---|---|---|---|---|---|---|---|---|---|---|---|---|
| | | | | | | | | | Solubility | Peak separation pattern | Carryover | pH | Osmotic pressure (mOsm) |
| Example 18 | Nonionic surfactant | Component A6 | EMULGEN LS-106 | Kao Corporation | Polyoxyethylene polyoxypropylene alkyl ether | 34 | 12.5 | 0.5% | ○○ | ○ | ○ | 7.34 | 101 |
| Example 19 | | Component A6 | EMULGEN LS-106 | Kao Corporation | Polyoxyethylene polyoxypropylene alkyl ether | 34 | 12.5 | 0.1% | ○○ | ○ | ○ | 7.34 | 101 |
| Example 20 | | Component A6 | EMULGEN LS-114 | Kao Corporation | Polyoxyethylene polyoxypropylene alkyl ether | 88 | 14 | 0.5% | ○ | ○ | ○ | 7.39 | 101 |
| Example 21 | | Component A6 | EMULGEN LS-114 | Kao Corporation | Polyoxyethylene polyoxypropylene alkyl ether | 88 | 14 | 0.1% | ○ | ○ | ○ | 7.39 | 101 |
| Example 22 | | Component A6 | EMULGEN MS-100 | Kao Corporation | Polyoxyethylene polyoxypropylene alkyl ether | 55 | 12.7 | 0.5% | ○○ | ○ | ○ | 7.36 | 102 |
| Example 23 | | Component A6 | EMULGEN MS-100 | Kao Corporation | Polyoxyethylene polyoxypropylene alkyl ether | 55 | 12.7 | 0.1% | ○○ | ○ | ○ | 7.36 | 102 |
| Comparative Example 44 | | | LATEMUL PD-420 | Kao Corporation | Polyoxyalkylene alkenyl ether | - | - | 0.5% | ○ | × | × | - | - |
| Comparative Example 45 | | | LATEMUL PD-430 | Kao Corporation | Polyoxyalkylene alkenyl ether | - | - | 0.5% | ○ | × | × | - | - |
| Comparative Example 46 | | | LATEMUL PD-450 | Kao Corporation | Polyoxyalkylene alkenyl ether | - | - | 0.5% | ○ | × | × | - | - |
| Comparative Example 47 | | | MEGA-8 | Dojindo Molecular Technologies, Inc. | n-octanoyl-N-methyl-D-glucamine | - | - | 0.5% | ○ | × | × | - | - |
| Example 24 | | Component A7 | MEGA-9 | Dojindo Molecular Technologies, Inc. | n-nonanoyl-N-methyl-D-glucamine | - | - | 0.5% | ○ | ○ | ○ | 7.28 | 117 |
| Comparative Example 48 | | | MEGA-10 | Dojindo Molecular Technologies, Inc. | n-decanoyl-N-methyl-D-glucamine | - | - | 0.5% | × | - | - | - | - |
| Example 25 | | Component A8 | - | Dojindo Molecular Technologies, Inc. | n-octyl-β-D-glucopyranoside | - | - | 0.5% | ○○ | ○ | ○ | 7.28 | 120 |
| Example 26 | | Component A9 | Saponin | NACALAI TESQUE, INC. | Soybean-derived saponin having structure of formula (9) | - | - | 0.5% | ○ | ○ | ○ | 6.32 | 123 |
| Comparative Example 49 | | | EMANON CH-40 | Kao Corporation | Polyoxyethylene hydrogenated castor oil (40E.O.) | - | - | 0.5% | × | - | - | - | - |
| Comparative Example 50 | | | EMANON CH-60K | Kao Corporation | Polyoxyethylene hydrogenated castor oil (60E.O.) | - | - | 0.5% | × | - | - | - | - |
| Comparative Example 51 | | | EMANON CH-80 | Kao Corporation | Polyoxyethylene hydrogenated castor oil (80E.O.) | - | - | 0.5% | × | - | - | - | - |
| Comparative Example 52 | | | RHEODOL 430V | Kao Corporation | Tetraoleic acid polyoxyethylene sorbit (30E.O.) | - | - | 0.5% | × | - | - | - | - |
| Comparative Example 53 | | | RHEODOL 440V | Kao Corporation | Tetraoleic acid polyoxyethylene sorbit (40E.O.) | - | - | 0.5% | × | - | - | - | - |
| Comparative Example 54 | | | RHEODOL 460V | Kao Corporation | Tetraoleic acid polyoxyethylene sorbit (60E.O.) | - | - | 0.5% | × | - | - | - | - |
| Comparative Example 55 | | | RHEODOL MS165V | Kao Corporation | Self-emulsifying glycerin monostearate | - | - | 0.5% | × | - | - | - | - |

| | | | Product name | Manufacturer | Name of component | Clouding point (°C) | HLB value | Content | Evaluation | | | | |
|---|---|---|---|---|---|---|---|---|---|---|---|---|---|
| | | | | | | | | | Solubility | Peak separation pattern | Carryover | pH | Osmotic pressure (mOsm) |
| Comparative Example 56 | Nonionic surfactant | | EMULGEN PP-290 | Kao Corporation | Polyoxyethylene polyoxypropylene glycol | - | - | 0.5% | ○ | × | × | - | - |
| Comparative Example 57 | | | Pluronic L-31 | ADEKA | Polyoxyethylene polyoxypropylene glycol | - | - | 0.5% | ○ | × | × | - | - |
| Comparative Example 58 | | | Pluronic L-71 | ADEKA | Polyoxyethylene polyoxypropylene glycol | - | - | 0.5% | × | - | - | - | - |
| Comparative Example 59 | | | Pluronic F-108 | ADEKA | Polyoxyethylene polyoxypropylene glycol | - | - | 0.5% | ○ | × | × | - | - |
| Comparative Example 60 | | | Pluronic P-85 | ADEKA | Polyoxyethylene polyoxypropylene glycol | - | - | 0.5% | ○ | × | × | - | - |
| Comparative Example 61 | | | Pluronic TR-704 | ADEKA | Ethylenediamine tetrapolyoxyethylene polyoxypropylene | - | - | 0.5% | ○ | × | × | - | - |
| Comparative Example 62 | | | Pluronic TR-913R | ADEKA | Ethylenediamine tetrapolyoxyethylene polyoxypropylene | - | - | 0.5% | ○ | × | × | - | - |
| Comparative Example 63 | Anionic surfactant | | - | - | Sodium lauryl sulfate | - | - | 0.5% | ○ | × | × | - | - |
| Comparative Example 64 | | | - | - | Sodium taurocholate | - | - | 0.5% | ○ | × | × | - | - |
| Comparative Example 65 | | | - | - | Sodium deoxycholate | - | - | 0.5% | ○ | × | × | - | - |
| Example 27 | Amphoteric surfactant | Component B1 | CHAPS | Dojindo Molecular Technologies, Inc. | 3-[(3-cholamidopropyl) dimethylammonio]propane sulfonate | - | - | 0.5% | ○ | ○ | ○ | 7.44 | 116 |
| Example 28 | | Component B2 | CHAPSO | Dojindo Molecular Technologies, Inc. | 3-[(3-cholamidopropyl) dimethylammonio]-2-hydroxypropane sulfonate | - | - | 0.5% | ○ | ○ | ○ | 7.41 | 110 |
| Comparative Example 66 | | | AMPHITOL 20BS | Kao Corporation | Lauryl dimethylamino acetic acid betaine | - | - | 0.5% | × | - | - | - | - |
| Comparative Example 67 | | | AMPHITOL 86B | Kao Corporation | Stearyl dimethylamino acetic acid betaine | - | - | 0.5% | × | - | - | - | - |
| Example 29 | | Component B3 | AMPHITOL 20N | Kao Corporation | Lauryl dimethylamine oxide | - | - | 0.5% | ○○ | ○ | ○ | 7.37 | 103 |
| Example 30 | | Component B3 | AMPHITOL 20N | Kao Corporation | Lauryl dimethylamine oxide | - | - | 0.1% | ○○ | ○ | ○ | 7.37 | 103 |
| Comparative Example 68 | | | AMPHITOL 20Y-B | Kao Corporation | 2-alkyl-N-carboxymethyl-N-hydroxyethyl imidazolinium betaine | - | - | 0.5% | × | - | - | - | - |
| Comparative Example 69 | - | | - | - | No surfactant added | - | - | 0% | - | × | × | - | - |

It can be understood that the reagents for measuring hemoglobins obtained in Examples 1 to 30 are capable of satisfactorily causing hemolysis and measuring hemoglobins with high accuracy.

(Reference Example A and Examples 31 to 38)

A reagent for measuring hemoglobins was prepared in the same manner as in Example 1 except that the compositions were changed to those shown in Table 7.

(5) Storage stability of reagent for measuring hemoglobins

The reagent for measuring hemoglobins obtained in Reference Example A and each reagent for measuring hemoglobins obtained in Examples 32, 34, 36 to 38 having a concentration of a surfactant of 0.5% by weight were respectively sealed in a glass vial and stored at 60°C for 7 days. After the storage, properties of the reagent for measuring hemoglobins were visually checked.

Generally, a reagent for measuring hemoglobins is stored at room temperature. In this evaluation item, the reagent for measuring hemoglobins was stored and evaluated under a temperature condition severer than that in a normal storage condition.

[Criteria for judging storage stability of reagent for measuring hemoglobins (60°C, 7 days)]

A: Phase separation does not occur
B: Phase separation occurs

(6) Storage stability of mixed solution

(6-1) Storage stability of mixed solution with whole blood

Whole blood (erythrocyte-containing sample) was prepared. Each reagent for measuring hemoglobins obtained in Reference Example A and Examples 31 to 36 was prepared. Whole blood was diluted 101-fold with the reagent for measuring hemoglobins to obtain a mixed solution. The obtained mixed solution was placed in a glass vial and stored at 4°C. Before the storage, and on the 1st day, the 2nd day, the 5th day, the 7th day, and the 9th day after the storage, the mixed solution was repeatedly measured 3 times under the measurement conditions described in "(2) Measurement by cation exchange liquid chromatography", and the value of hemoglobin A1c and the variation in the value of the hemoglobin A1c were determined from the average.

Value of hemoglobin A1c (%) = peak area of hemoglobin A1c/total peak area of hemoglobins

Variation in value of hemoglobin A1c (%) = value of hemoglobin A1c before storage - value of hemoglobin A1c after storage

Fig. 6(a) is a figure showing the relationship between the number of storage days and the value of hemoglobin A1c in a mixed solution with whole blood. Fig. 6(b) is a figure showing the relationship between the number of storage days and the variation in the value of hemoglobin A1c in a mixed solution with whole blood.

[Criteria for judging mixed solution with whole blood]

○: For the variation in the value of hemoglobin A1c on the 5th day, the absolute value of the difference from the value of Reference Example A is 0.2% or less

Δ: For the variation in the value of hemoglobin A1c on the 5th day, the absolute value of the difference from the value of Reference Example A is more than 0.2% and 0.4% or less.

ΔΔ: For the variation in the value of hemoglobin A1c on the 5th day, the absolute value of the difference from the value of Reference Example A is more than 0.4% and 0.7% or less.
×: For the variation in the value of hemoglobin A1c on the 5th day, the absolute value of the difference from the value of Reference Example A is more than 0.7%.

(6-2) Storage stability of mixed solution with hemoglobin A1c substance for measurement control (low concentration) (IRC-L)

A hemoglobin A1c substance for measurement control having a value of hemoglobin A1c of 5.8% (low concentration) (IRC-L) was prepared. Each reagent for measuring hemoglobins obtained in Reference Example A and Examples 31 to 36 was prepared. The hemoglobin A1c substance for measurement control (low concentration) (IRC-L) was diluted 101-fold with the reagent for measuring hemoglobins to obtain a mixed solution. The obtained mixed solution was placed in a glass vial and stored at 4°C. Before the storage, and on the 1st day, the 2nd day, the 3rd day, the 9th day, and the 14th day after the storage, the mixed solution was repeatedly measured 3 times under the measurement conditions in "(2) Measurement by cation exchange liquid chromatography", and the value of hemoglobin A1c and the variation in the value of the hemoglobin A1c were determined from the average in the same manner as above.
Fig. 7(a) is a figure showing the relationship between the number of storage days and the value of hemoglobin A1c in a mixed solution with a hemoglobin A1c substance for measurement control (low concentration) (IRC-L). Fig. 7(b) is a figure showing the relationship between the number of storage days and the variation in the value of hemoglobin A1c in a mixed solution with a hemoglobin A1c substance for measurement control (low concentration) (IRC-L) .

[Criteria for judging storage stability of mixed solution with hemoglobin A1c substance for measurement control (low concentration) (IRC-L)]

○: For the variation in the value of hemoglobin A1c on the 14th day, the absolute value of the difference from the value of Reference Example A is 0.2% or less
Δ: For the variation in the value of hemoglobin A1c on the 14th day, the absolute value of the difference from the value of Reference Example A is more than 0.2% and 0.4% or less.
ΔΔ: For the variation in the value of hemoglobin A1c on the 14th day, the absolute value of the difference from the value of Reference Example A is more than 0.4% and 0.7% or less.
×: For the variation in the value of hemoglobin A1c on the 14th day, the absolute value of the difference from the value of Reference Example A is more than 0.7%.

(6-3) Storage stability of mixed solution with hemoglobin A1c substance for measurement control (high concentration) (IRC-H)

The value of hemoglobin A1c and the variation in the value of hemoglobin A1c were determined in the same manner as in "(6-2) Storage stability of mixed solution with hemoglobin A1c substance for measurement control (low concentration) (IRC-L)" except that the hemoglobin A1c substance for measurement control (low concentration) (IRC-L) was changed to the hemoglobin A1c substance for measurement control having a value of hemoglobin A1c of 10.4% (high concentration) (IRC-H).
Fig. 8(a) is a figure showing the relationship between the number of storage days and the value of hemoglobin A1c in a mixed solution with a hemoglobin A1c substance for measurement control (high concentration) (IRC-H). Fig. 8(b) is a figure showing the relationship between the number of storage days and the variation in the value of hemoglobin A1c in a mixed solution with a hemoglobin A1c substance for measurement control (high concentration) (IRC-H).

[Criteria for judging storage stability of mixed solution with hemoglobin A1c substance for measurement control (high concentration) (IRC-H)]

○: For the variation in the value of hemoglobin A1c on the 14th day, the absolute value of the difference from the value of Reference Example A is 0.25% or less
Δ: For the variation in the value of hemoglobin A1c on the 14th day, the absolute value of the difference from the value of Reference Example A is more than 0.25% and 0.45% or less
ΔΔ: For the variation in the value of hemoglobin A1c on the 14th day, the absolute value of the difference from the

value of Reference Example A is more than 0.45% and 0.75% or less
×: For the variation in the value of hemoglobin A1c on the 14th day, the absolute value of the difference from the value of Reference Example A is more than 0.75%

The compositions and results are shown in Tables 7 below.

[Table 7]

| | Product name | Name of component | | Reference Example A | Example 31 | Example 32 | Example 33 | Example 34 | Example 35 | Example 36 | Example 37 | Example 38 |
|---|---|---|---|---|---|---|---|---|---|---|---|---|
| | | Potassium dihydrogen phosphate | % by weight | 0.013 | 0.013 | 0.013 | 0.013 | 0.013 | 0.013 | 0.013 | 0.013 | 0.013 |
| | | Dipotassium hydrogen phosphate | % by weight | 0.052 | 0.052 | 0.052 | 0.052 | 0.052 | 0.052 | 0.052 | 0.052 | 0.052 |
| | | Sodium chloride | % by weight | 0.233 | 0.233 | 0.233 | 0.233 | 0.233 | 0.233 | 0.233 | 0.233 | 0.233 |
| | | Sodium azide | % by weight | 0.02 | 0.02 | 0.02 | 0.02 | 0.02 | 0.02 | 0.02 | 0.02 | 0.02 |
| Component X | Triton X-100 | Polyoxyethylene octyl phenyl ether (10 E.O.) | % by weight | 0.1 | | | | | | | | |
| Component A1 | EMULGEN 120 | Polyoxyethylene lauryl ether (12 E.O.) | % by weight | | 0.1 | 0.5 | | | | | | |
| Component A1 | EMULGEN 220 | Polyoxyethylene cetyl ether (13 E.O.) | % by weight | | | | 0.1 | 0.5 | | | | |
| Component A3 | EMULGEN 420 | Polyoxyethylene oleyl ether (13 E.O.) | % by weight | | | | | | 0.1 | 0.5 | | |
| Component A6 | EMULGEN LS-114 | Polyoxyethylene polyoxypropylene alkyl ether | % by weight | | | | | | | | 0.5 | |
| Component A6 | EMULGEN MS-100 | Polyoxyethylene polyoxypropylene alkyl ether | % by weight | | | | | | | | | 0.5 |
| | | Water | % by weight | Remainder | Remainder | Remainder | Remainder | Remainder | Remainder | Remainder | Remainder | Remainder |
| | | Total | % by weight | 100 | 100 | 100 | 100 | 100 | 100 | 100 | 100 | 100 |

(continued)

| | Product name | Name of component | | Reference Example A | Example 31 | Example 32 | Example 33 | Example 34 | Example 35 | Example 36 | Example 37 | Example 38 |
|---|---|---|---|---|---|---|---|---|---|---|---|---|
| Evaluation | Storage stability of reagent for measuring hemoglobins (60°C, 7 days) | | Judgement | A | - | A | - | A | - | A | B | B |
| | Storage stability of mixed solution | Mixed solution with whole blood (4°C, 5 days) | Judgement | - | ○ | ○ | ○ | Δ | Δ | ΔΔ | - | - |
| | | Mixed solution with hemoglobin A1c substance for measurement control (low concentration) (IRC-L) (4°C, 14 days) | Judgement | - | ○ | Δ | ○ | Δ | ○ | ○ | - | - |
| | | Mixed solution with hemoglobin A1c substance for measurement control (high concentration) (IRC-H) (4°C, 14 days) | Judgement | - | Δ | ΔΔ | ○ | ΔΔ | ○ | ○ | - | - |

Each reagent for measuring hemoglobins obtained in Examples 31 to 36 successfully increased its storage stability compared with each reagent for measuring hemoglobins obtained in Examples 37 and 38. Each reagent for measuring hemoglobins obtained in Examples 31 to 36 also successfully increased the storage stability of a mixed solution obtained by mixing the reagent and the substance to be tested. Each reagent for measuring hemoglobins obtained in Examples 31 to 36 can measure hemoglobins with high accuracy even after the mixed solution obtained by mixing the reagent for measuring hemoglobins and the substance to be tested is stored for a long time.

(7) Column durability

Whole blood (erythrocyte-containing sample), a hemoglobin A1c substance for measurement control having a value of hemoglobin A1c of 10.4% (high concentration) (IRC-H), and each reagent for measuring hemoglobins obtained in Reference Example A and Example 32 were prepared. Whole blood was diluted 101-fold with the reagent for measuring hemoglobins to obtain Liquid (1). The hemoglobin A1c substance for measurement control was diluted 101-fold with the reagent for measuring hemoglobins to obtain Liquid (2). Liquid (1) is a measurement sample for applying a load to the column, and Liquid (2) is a measurement sample for checking the variation in the value of hemoglobin A1c and the variation in the number of theoretical plates. The obtained Liquid (1) and Liquid (2) were measured according to the following procedures under the following conditions.

First, the obtained Liquid (2) was measured 3 times. Then, every time the obtained Liquid (1) was passed through the column multiple times (tens of times to 200 times), Liquid (2) was measured 3 times. Liquid (1) and Liquid (2) were repeatedly measured according to the above procedure until the number of samples loaded on the column reached about 3000.

Cation exchange chromatography conditions:

HPLC instrument: glycohemoglobin analyzer ("HA-8160VP" manufactured by ARKRAY, Inc.)
Cation exchange column: COLUMN UNIT HSVI-VP (manufactured by ARKRAY, Inc.)
Eluent A: ELUENT 60A-VP/TP (manufactured by ARKRAY, Inc.)
Eluent B: ELUENT 60B-VP/TP (manufactured by ARKRAY, Inc.)
Eluent C: ELUENT 60C-VP (manufactured by ARKRAY, Inc.)

The measurement was performed by the measurement method installed in the HPLC instrument.

Fig. 9(a) is a figure showing the relationship between the number of samples loaded on the column and the variation in the value of hemoglobin Ale. Fig. 9(b) is a figure showing the relationship between the number of samples loaded on the column and the variation in the number of theoretical plates.

In Fig. 9(a), the vertical axis shows the difference between the value of hemoglobin A1c in Liquid (2) measured first and the value of hemoglobin A1c in Liquid (2) measured at each number of samples loaded (value of hemoglobin A1c in Liquid (2) measured first - value of hemoglobin A1c in Liquid (2) measured at each number of samples loaded). In Fig. 9(b), the vertical axis shows the difference between the number of theoretical plates in Liquid (2) measured first and the number of theoretical plates in Liquid (2) measured at each number of samples loaded (number of theoretical plates in Liquid (2) measured first - number of theoretical plates in Liquid (2) measured at each number of samples loaded).

The reagent for measuring hemoglobins obtained in Example 32 was superior to the reagent for measuring hemoglobins obtained in Reference Example A in column durability.

**Claims**

**1.** A reagent for measuring hemoglobins used for measuring hemoglobins by cation exchange liquid chromatography, comprising:

a nonionic surfactant or an amphoteric surfactant,
the nonionic surfactant being Component A1 below, Component A2 below, Component A3 below, Component A4 below, Component A5 below, Component A6 below, Component A7 below, Component A8 below, or Component A9 below, and
the amphoteric surfactant being Component B1 below, Component B2 below, or Component B3 below:

Component A1: Polyoxyethylene alkyl ether having an average number of moles of an oxyethylene group added of 8 or more and 20 or less, and a carbon number of an alkyl group of 12 or more and 17 or less
Component A2: Polyoxyethylene stearyl ether having an average number of moles of an oxyethylene group added of 40 or more and 60 or less

Component A3: Polyoxyethylene oleyl ether having an average number of moles of an oxyethylene group added of 9 or more and 15 or less
Component A4: Polyoxyethylene-sec-alkyl ether having an average number of moles of an oxyethylene group added of 6 or more and 10 or less, and a carbon number of an alkyl group of 11 or more and 15 or less
Component A5: Polyoxyethylene tridecyl ether having an average number of moles of an oxyethylene group added of 15
Component A6: Polyoxyethylene polyoxypropylene alkyl ether having an HLB value of 11 or more and 15 or less
Component A7: n-nonanoyl-N-methyl-D-glucamine
Component A8: n-octyl-β-D-glucopyranoside
Component A9: Saponin
Component B1: 3-[(3-cholamidopropyl)dimethylammonio]propane sulfonate
Component B2: 3-[(3-cholamidopropyl)dimethylammonio]-2-hydroxypropane sulfonate
Component B3: Lauryl dimethylamine oxide.

**2.** The reagent for measuring hemoglobins according to claim 1, comprising the nonionic surfactant,
the nonionic surfactant including the Component A1, the Component A2, the Component A3, the Component A4, the Component A5, or the Component A6,
the Component A1 being polyoxyethylene lauryl ether having an average number of moles of an oxyethylene group added of 9, 12, or 19, or polyoxyethylene cetyl ether having an average number of moles of an oxyethylene group added of 13 or 20,
the Component A2 being polyoxyethylene stearyl ether having an average number of moles of an oxyethylene group added of 50,
the Component A3 being polyoxyethylene oleyl ether having an average number of moles of an oxyethylene group added of 9 or 13,
the Component A4 being polyoxyethylene-sec-alkyl ether having an average number of moles of an oxyethylene group added of 7 or 9 and a carbon number of an alkyl group of 11 or more and 15 or less, and
the Component A6 being polyoxyethylene polyoxypropylene alkyl ether having an HLB value of 12.5, 12.7, or 14.0.

**3.** The reagent for measuring hemoglobins according to claim 1, comprising the nonionic surfactant,
the nonionic surfactant including the Component A1 or the Component A3,
the Component A1 being polyoxyethylene alkyl ether having an average number of moles of an oxyethylene group added of 10 or more and 15 or less, and a carbon number of an alkyl group of 12 or more and 17 or less, and
the Component A3 being polyoxyethylene oleyl ether having an average number of moles of an oxyethylene group added of 10 or more and 15 or less.

**4.** The reagent for measuring hemoglobins according to any one of claims 1 to 3,
the Component A1 being polyoxyethylene lauryl ether having an average number of moles of an oxyethylene group added of 12, or polyoxyethylene cetyl ether having an average number of moles of an oxyethylene group added of 13, and
the Component A3 being polyoxyethylene oleyl ether having an average number of moles of an oxyethylene group added of 13.

**5.** The reagent for measuring hemoglobins according to any one of claims 1 to 4, comprising the nonionic surfactant, the reagent for measuring hemoglobins having a content of the nonionic surfactant of 0.01% by weight or more and 1.0% by weight or less.

**6.** The reagent for measuring hemoglobins according to any one of claims 1 to 5, comprising the amphoteric surfactant, the reagent for measuring hemoglobins having a content of the amphoteric surfactant of 0.01% by weight or more and 1.0% by weight or less.

**7.** A method for measuring hemoglobins, comprising the steps of:

mixing an erythrocyte-containing sample and the reagent for measuring hemoglobins according to any one of claims 1 to 6 to obtain a mixed solution; and
measuring the mixed solution by cation exchange liquid chromatography.

[FIG. 1.]

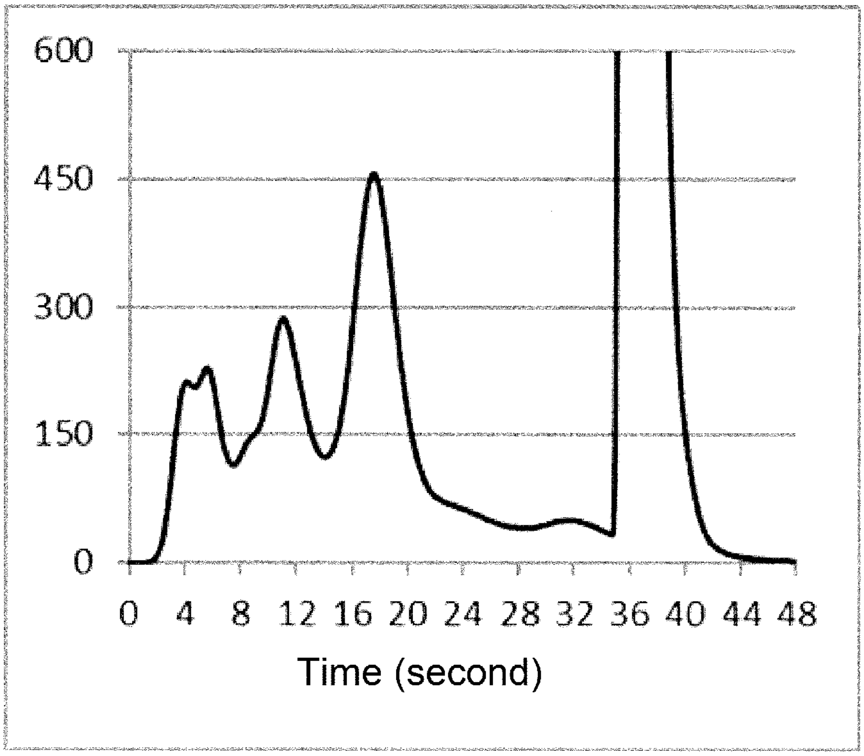
600
450
300
150
0
0 4 8 12 16 20 24 28 32 36 40 44 48
Time (second)

[FIG. 2.]

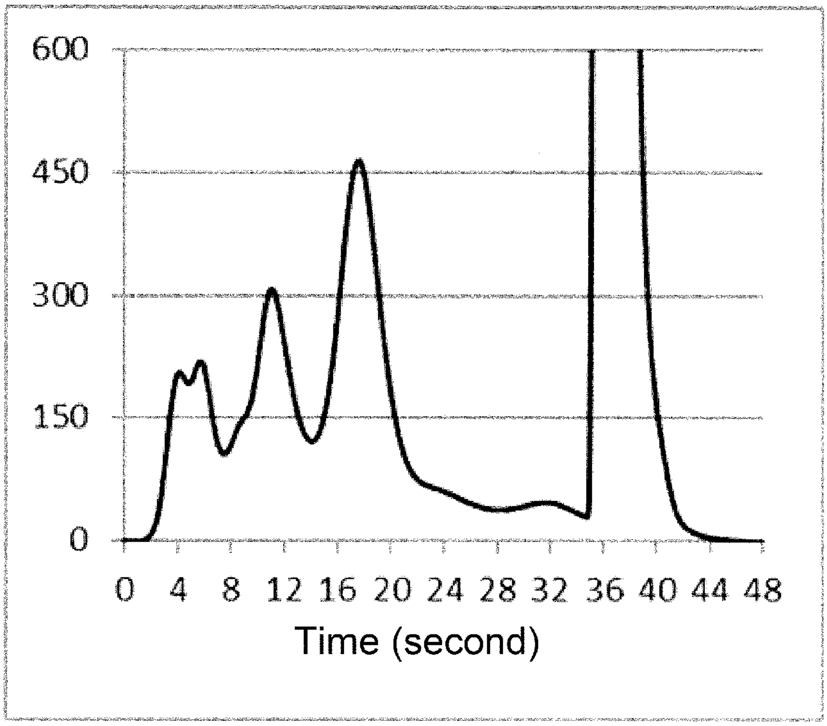
600
450
300
150
0
0 4 8 12 16 20 24 28 32 36 40 44 48
Time (second)

[FIG. 3.]

(a)

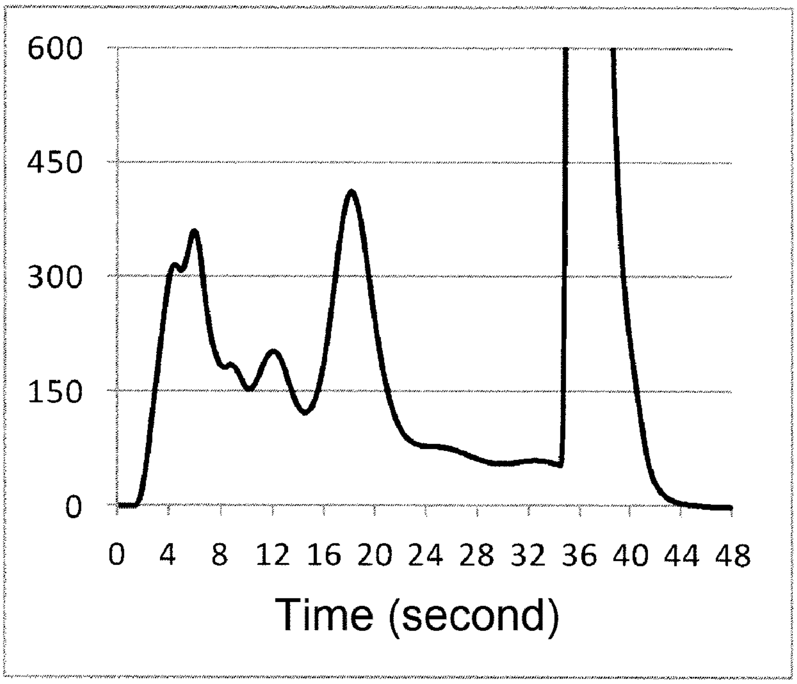
600
450
300
150
0
0 4 8 12 16 20 24 28 32 36 40 44 48
Time (second)

(b)

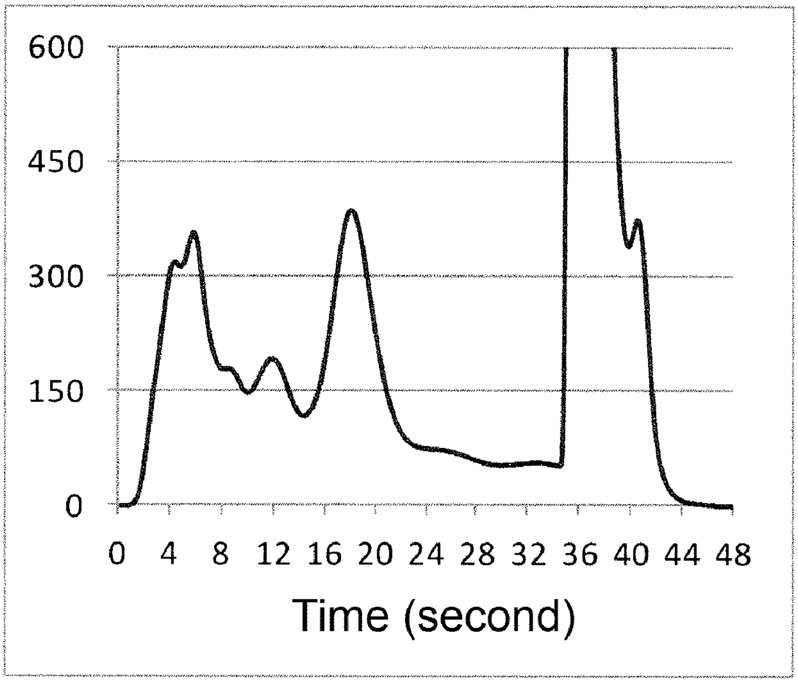
600
450
300
150
0
0 4 8 12 16 20 24 28 32 36 40 44 48
Time (second)

[FIG. 4.]

(a)

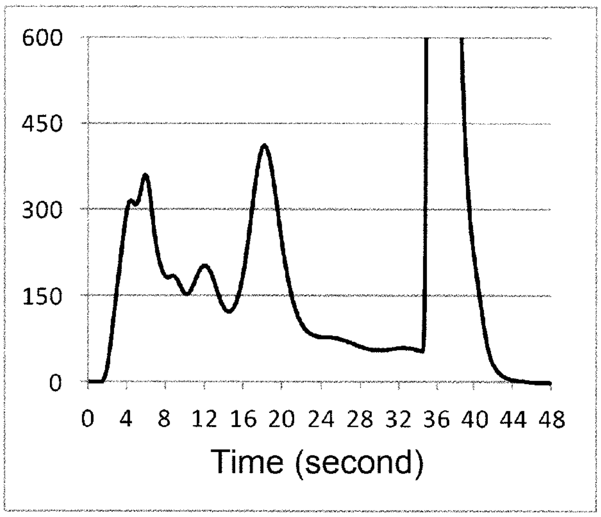
600
450
300
150
0
0 4 8 12 16 20 24 28 32 36 40 44 48
Time (second)

(b)

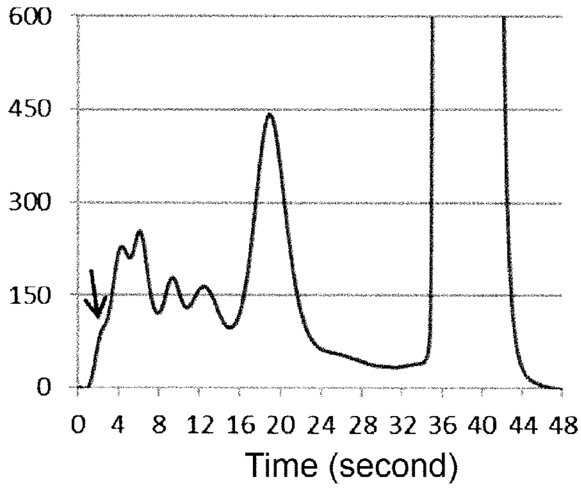
600
450
300
150
0
0 4 8 12 16 20 24 28 32 36 40 44 48
Time (second)

[FIG. 5.]

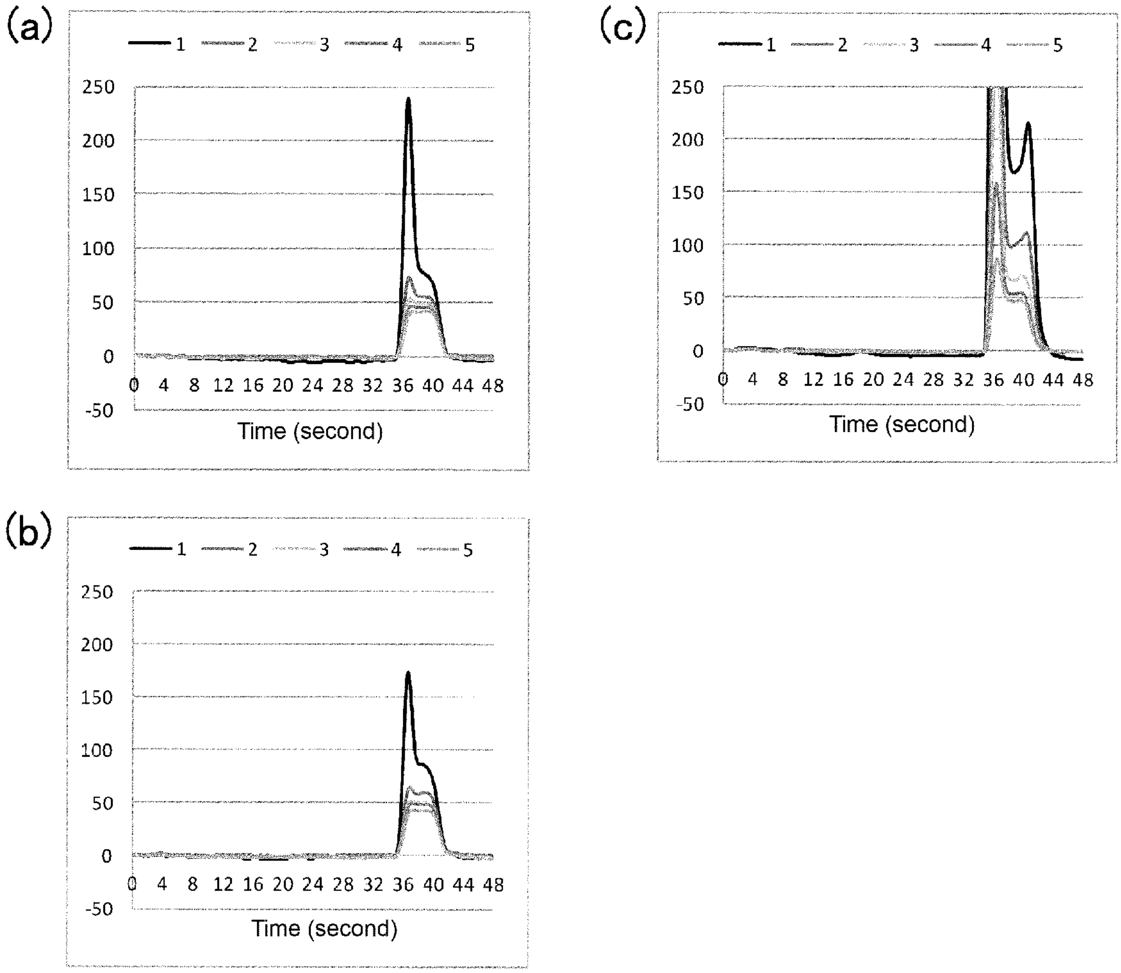
(a)
1 2 3 4 5
250
200
150
100
50
0
-50
0 4 8 12 16 20 24 28 32 36 40 44 48
Time (second)
(b)
1 2 3 4 5
250
200
150
100
50
0
-50
0 4 8 12 16 20 24 28 32 36 40 44 48
Time (second)
(c)
1 2 3 4 5
250
200
150
100
50
0
-50
0 4 8 12 16 20 24 28 32 36 40 44 48
Time (second)

[FIG. 6.]

(a)

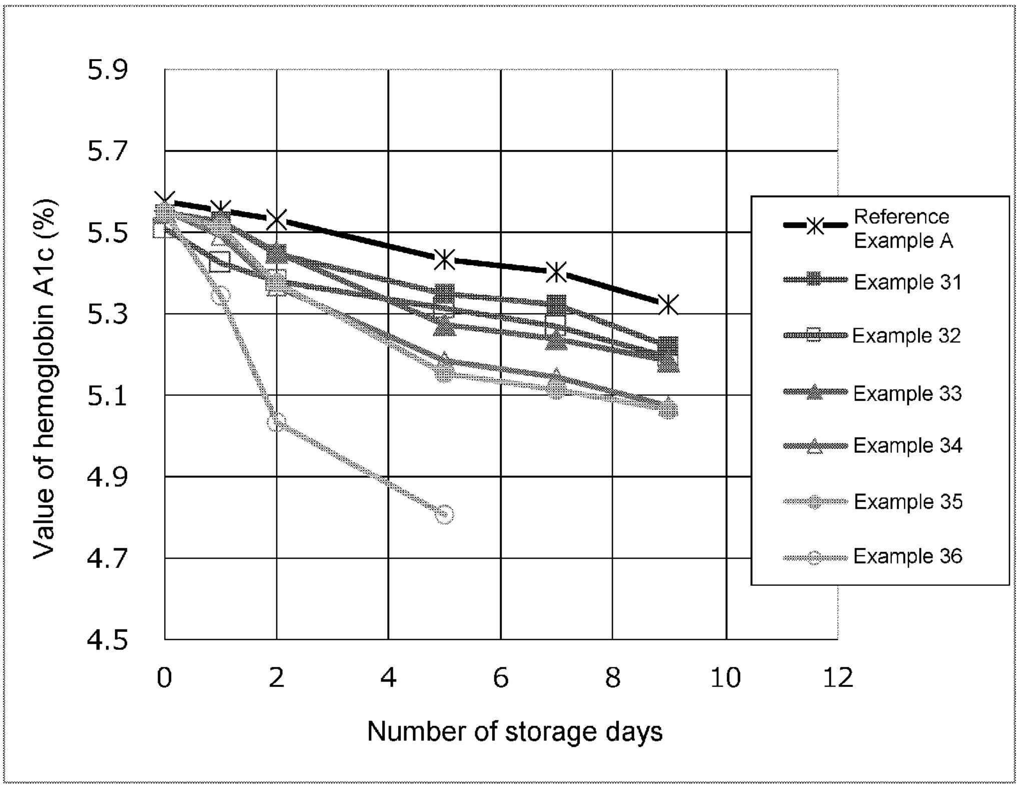
5.9
5.7
5.5
5.3
5.1
4.9
4.7
4.5
Value of hemoglobin A1c (%)
0
2
4
6
8
10
12
Number of storage days
Reference Example A
Example 31
Example 32
Example 33
Example 34
Example 35
Example 36

(b)

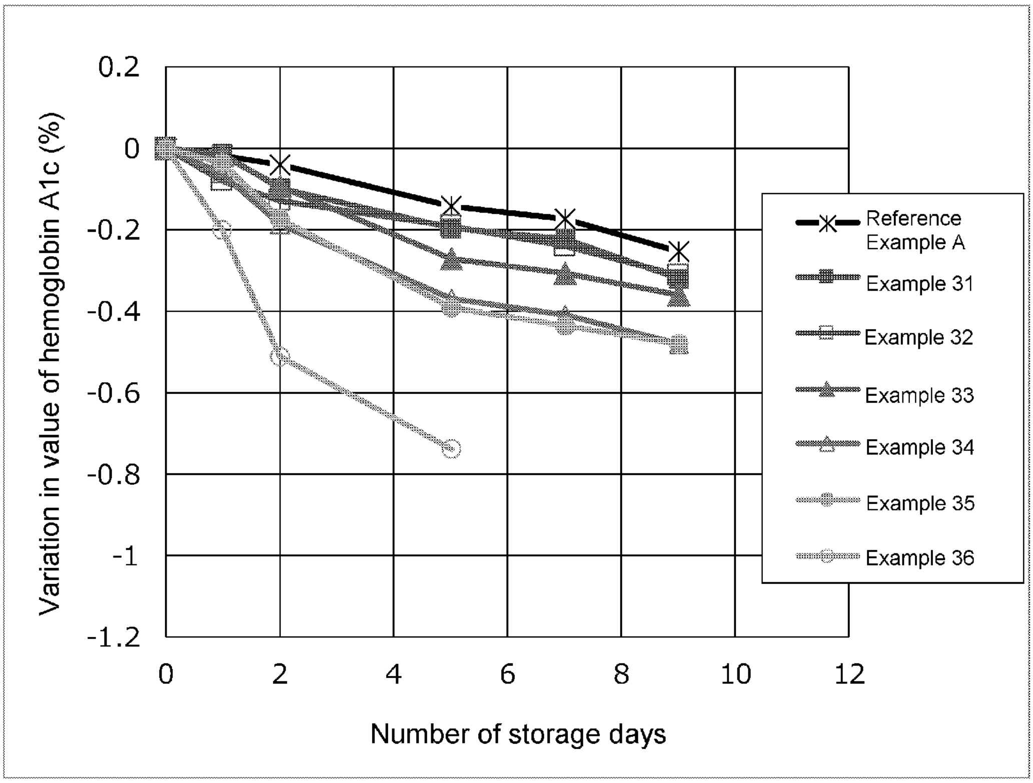
0.2
0
-0.2
-0.4
-0.6
-0.8
-1
-1.2
Variation in value of hemoglobin A1c (%)
0
2
4
6
8
10
12
Number of storage days
Reference Example A
Example 31
Example 32
Example 33
Example 34
Example 35
Example 36

[FIG. 7.]

(a)

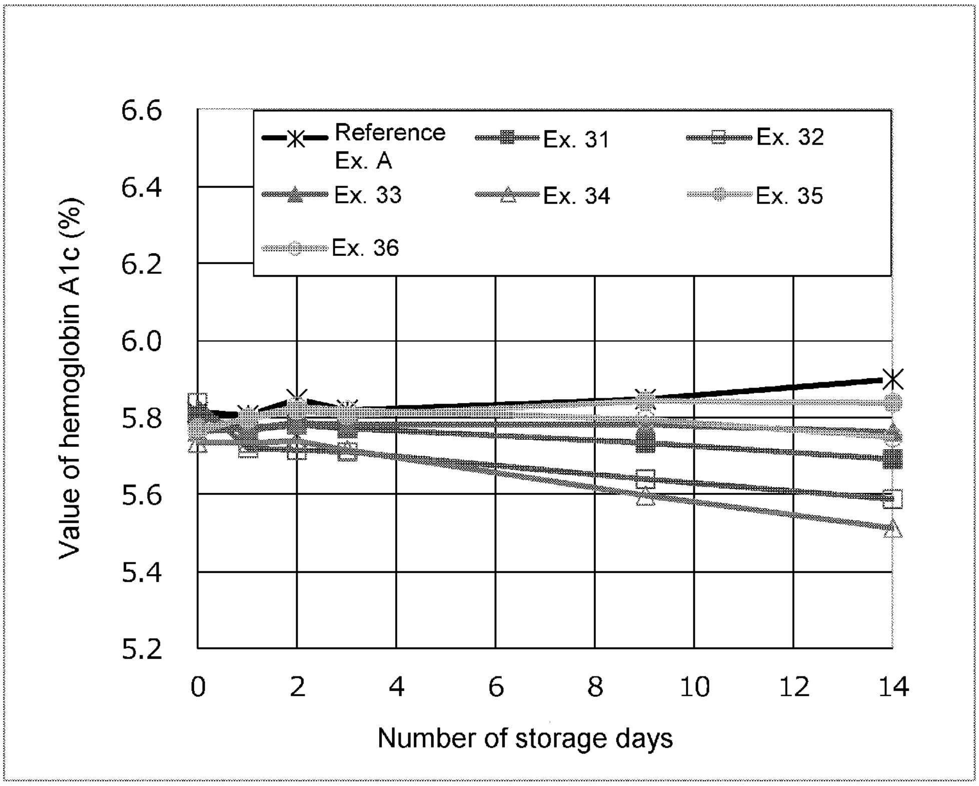

Value of hemoglobin A1c (%)
6.6
6.4
6.2
6.0
5.8
5.6
5.4
5.2
0
2
4
6
8
10
12
14
Number of storage days
Reference Ex. A
Ex. 31
Ex. 32
Ex. 33
Ex. 34
Ex. 35
Ex. 36


(b)

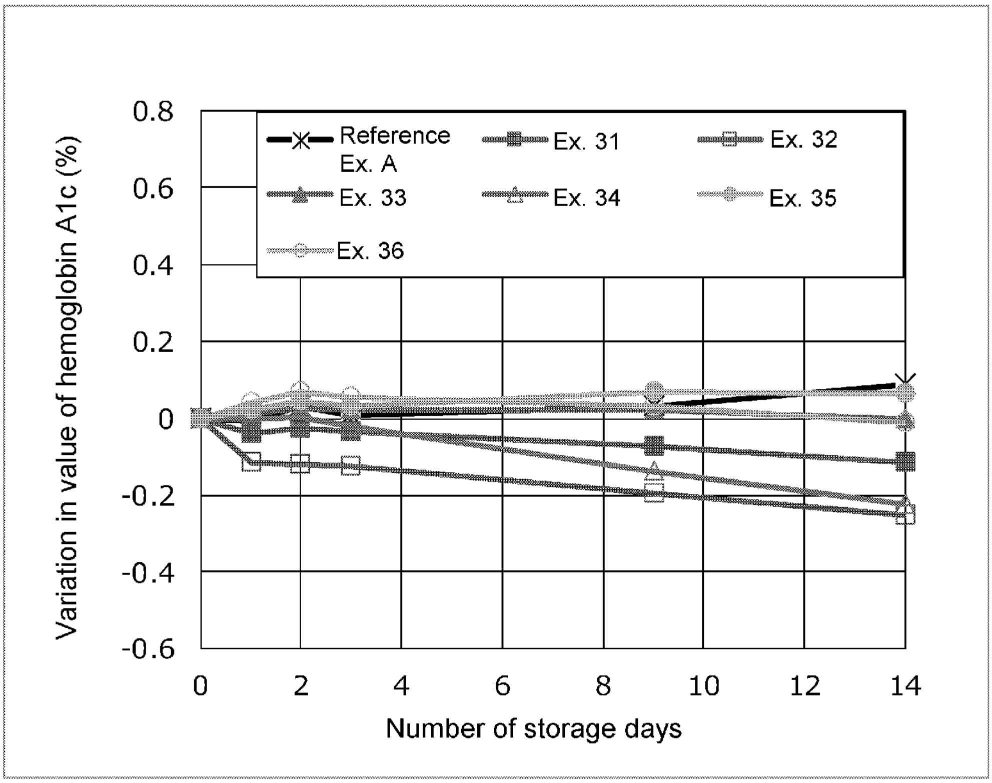

Variation in value of hemoglobin A1c (%)
0.8
0.6
0.4
0.2
0
-0.2
-0.4
-0.6
0
2
4
6
8
10
12
14
Number of storage days
Reference Ex. A
Ex. 31
Ex. 32
Ex. 33
Ex. 34
Ex. 35
Ex. 36

[FIG. 8.]

(a)

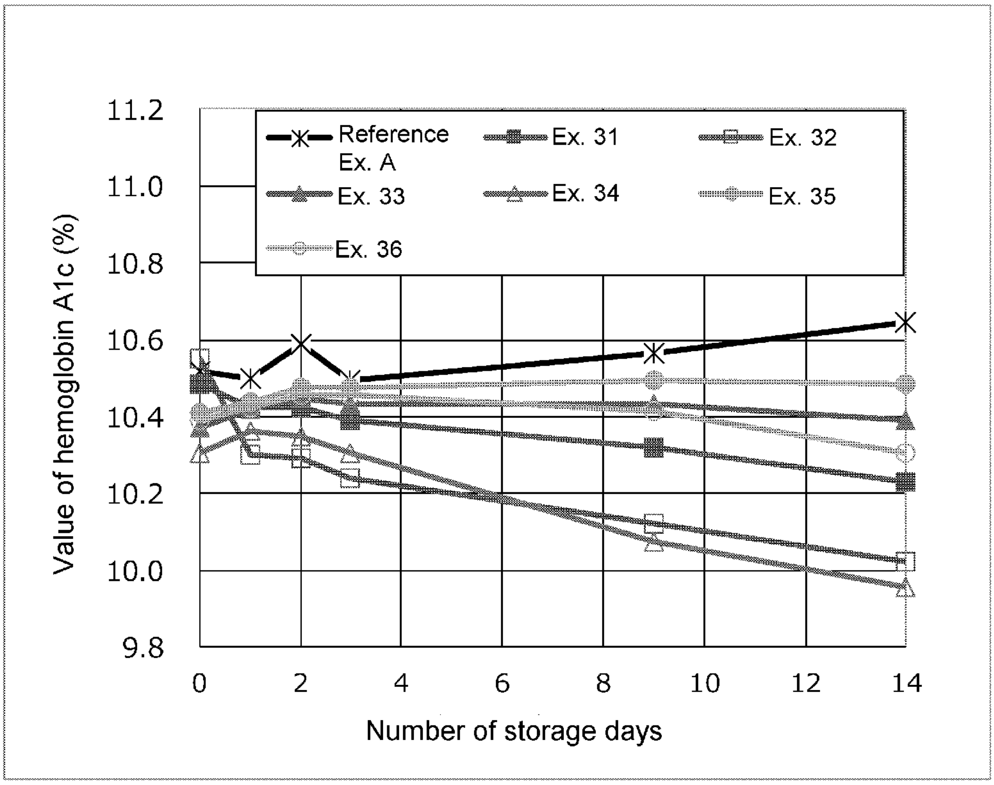
11.2
11.0
10.8
10.6
10.4
10.2
10.0
9.8
0
2
4
6
8
10
12
14
Value of hemoglobin A1c (%)
Number of storage days
Reference Ex. A
Ex. 31
Ex. 32
Ex. 33
Ex. 34
Ex. 35
Ex. 36

(b)

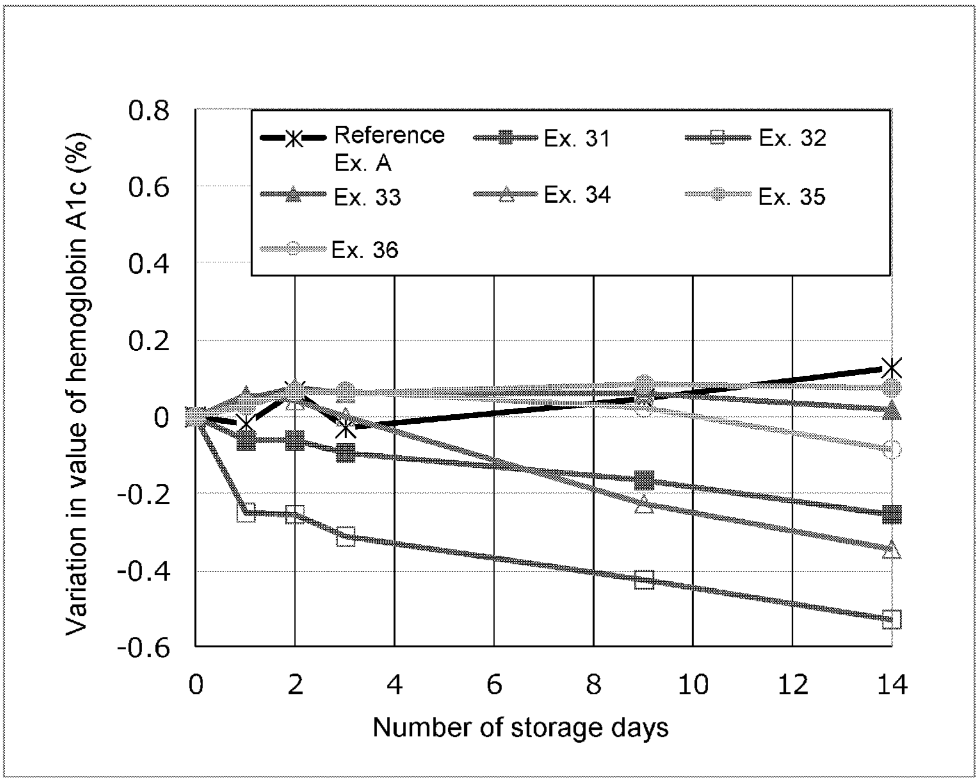
0.8
0.6
0.4
0.2
0
-0.2
-0.4
-0.6
0
2
4
6
8
10
12
14
Variation in value of hemoglobin A1c (%)
Number of storage days
Reference Ex. A
Ex. 31
Ex. 32
Ex. 33
Ex. 34
Ex. 35
Ex. 36

[FIG. 9.]

(a)

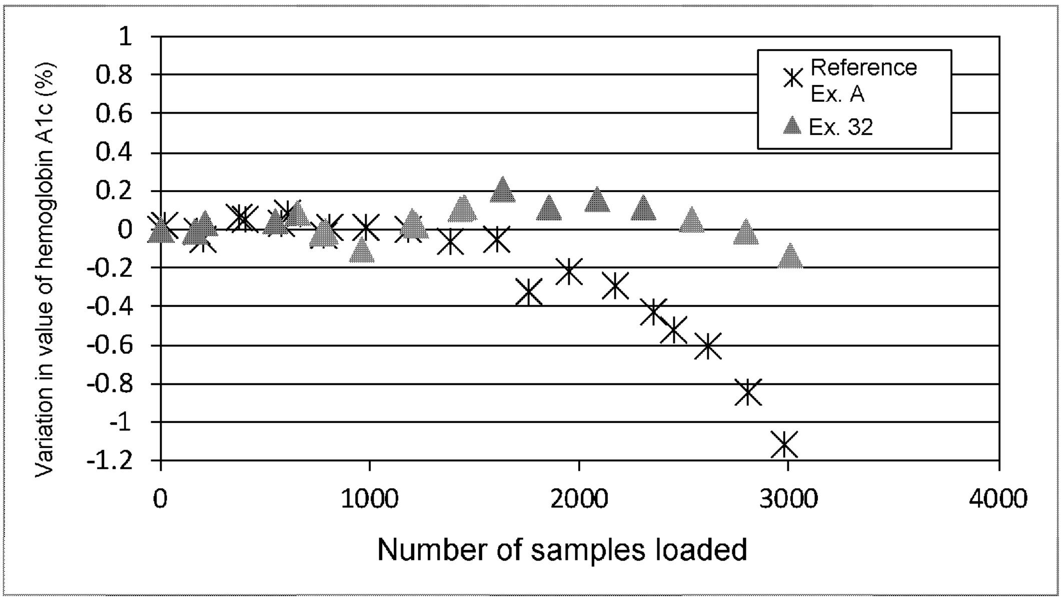
Variation in value of hemoglobin A1c (%)
1
0.8
0.6
0.4
0.2
0
-0.2
-0.4
-0.6
-0.8
-1
-1.2
0
1000
2000
3000
4000
Number of samples loaded
Reference Ex. A
Ex. 32

(b)

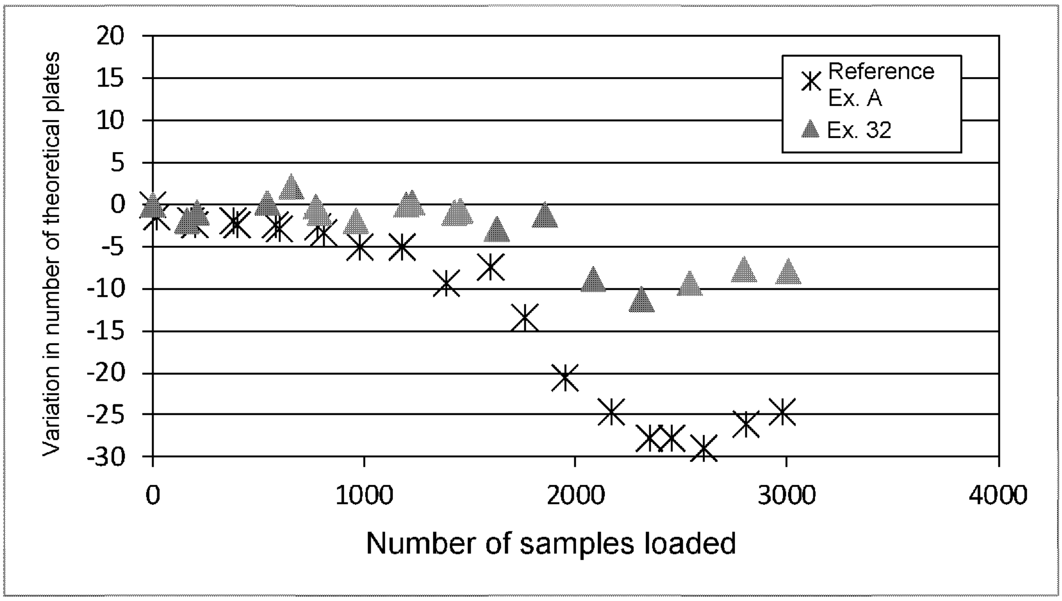
Variation in number of theoretical plates
20
15
10
5
0
-5
-10
-15
-20
-25
-30
0
1000
2000
3000
4000
Number of samples loaded
Reference Ex. A
Ex. 32

**INTERNATIONAL SEARCH REPORT**

International application No. PCT/JP2019/034972

A. CLASSIFICATION OF SUBJECT MATTER
Int.Cl. G01N30/06(2006.01)i, G01N30/88(2006.01)i

According to International Patent Classification (IPC) or to both national classification and IPC

B. FIELDS SEARCHED

Minimum documentation searched (classification system followed by classification symbols)
Int.Cl. G01N30/02, G01N30/06, G01N30/88, G01N33/49, G01N33/72, G01N1/28, G01N1/38

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII)

C. DOCUMENTS CONSIDERED TO BE RELEVANT

| Category* | Citation of document, with indication, where appropriate, of the relevant passages | Relevant to claim No. |
|---|---|---|
| Y | JP 2003-344372 A (ARKRAY, INC.) 03 December 2003, claims, paragraphs [0001], [0020], [0028]-[0029] (Family: none) | 1-2, 5, 7 |
| Y | JP 2012-27014 A (ARKRAY, INC., SEKISUI MEDICAL CO., LTD.) 09 February 2012, claims, paragraph [0019] & US 2012/0015388 A1, claims, paragraph [0066] & EP 2400034 A1 & CN 102297801 A | 1, 4-5, 7 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 November 2019 (12.11.2019) | 26 November 2019 (26.11.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2019/034972

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2018/008447 A1 (TOSOH CORP.) 11 January 2018, paragraphs [0003], [0015], [0023] & JP 2018-4606 A & EP 3483603 A1, paragraphs [0003], [0015], [0027] | 1-2, 4-5, 7 |
| A | JP 1-277755 A (SEKISUI CHEMICAL CO., LTD.) 08 November 1989, claims, examples 1, 3-2 (Family: none) | 1-7 |
| A | JP 2007-163182 A (FUJIFILM CORP.) 28 June 2007, claims, paragraphs [0025], [0046] (Family: none) | 1-7 |
| A | JP 2014-25918 A (ARKRAY, INC.) 06 February 2014, claims, paragraph [0097] no. 2, 4, 13 & US 2013/0344480 A1, claims, table 1 & EP 2677317 A1 & CN 103513021 A | 1-7 |
| A | WO 2009/116268 A1 (SEKISUI MEDICAL CO., LTD.) 24 September 2009, claims, paragraph [0050] & US 2011/0091993 A1, claims, paragraphs [0108]-[0110] & EP 2256497 A1 | 1-7 |
| A | JP 2013-36959 A (TOYOBO CO., LTD.) 21 February 2013, claims, paragraph [0042], experimental no. 5, 15-16 (Family: none) | 1-7 |
| A | JP 2014-235076 A (HITACHI HIGH-TECHNOLOGIES CORP.) 15 December 2014, claims, paragraph [0038] (Family: none) | 1-7 |
| A | JP 2004-77457 A (ASAHI KASEI PHARMA CORPORATION) 11 March 2004, paragraph [0091] (Family: none) | 1-7 |
| A | WO 2012/153773 A1 (SEKISUI MEDICAL CO., LTD.) 15 November 2012, claims, paragraph [0047] & US 2014/0080158 A1, claims, paragraphs [0069]-[0074] & EP 2708893 A1 & CA 2835068 A1 & CN 103502813 A & KR 10-2014-0033365 A | 1-7 |
| A | JP 9-511575 A (ABBOTT LABORATORIES) 18 November 1997, claims, example 1 & US 5612223 A & WO 1995/024651 A1, claims, example 1 & EP 749583 A1 & AU 1984295 A & CA 2183558 A1 | 1-7 |

Form PCT/ISA/210 (continuation of second sheet) (January 2015)

**INTERNATIONAL SEARCH REPORT**

International application No.
PCT/JP2019/034972

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.
2. ☒ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT

International application No. PCT/JP2019/034972

<Continuation of Box No. III>

(Invention 1) Invention including any one among a component A1 to a component A6 in claims 1-7

The invention understood by selecting the first option of claim 1 (hereinafter, referred to as "the first described invention") includes a nonionic surfactant, wherein the nonionic surfactant is a component A1, which is a reagent for measuring hemoglobin used to measure hemoglobin by a cation exchange liquid chromatography.

In addition, the invention including any one among a component A2 to a component A6 in claim 1 and the first described invention share the common technical feature of including a nonionic surfactant, wherein the nonionic surfactant is a an aliphatic hydrocarbon ether having a polyoxyethylene structure, which is a reagent for measuring hemoglobin used to measure hemoglobin by a cation exchange liquid chromatography, and thus is classified as invention 1.

(Invention 2) Invention including a component A7 in claims 1-7

In claims 1-7, the invention including a component A7 and the invention classified as invention 1 share the common technical feature of a reagent which includes a nonionic surfactant and is for measuring hemoglobin used to measure hemoglobin by a cation exchange liquid chromatography.

However, said technical feature does not make a contribution over the prior art in light of the disclosure of document 2 exemplified in paragraph [0005] of the present description and thus cannot be considered a special technical feature.

In addition, there are no other identical or corresponding special technical features between these inventions.

Furthermore, the invention including a component A7 in claims 1-7 is not substantially identical or equivalent to any of the claims classified as invention 1.

Therefore, the invention including a component A7 in claims 1-7 cannot be classified as invention 1.

(Invention 3) Invention including a component A8 in claims 1-7

The invention including a component A8 in claims 1-7 is the same as invention 2.

Furthermore, the invention including a component A7 in claims 1-7 is not substantially identical or equivalent to any invention classified as invention 1 or 2.

Therefore, the invention including a component A8 in claims 1-7 cannot be classified as invention 1 or 2.

(Invention 4) Invention including a component A9 in claims 1-7

The invention including a component A9 in claims 1-7 is the same as invention 2.

Furthermore, the invention including a component A9 in claims 1-7 is not substantially identical or equivalent to any invention classified as inventions 1-3.

Therefore, the invention including a component A9 in claims 1-7 cannot be classified as inventions 1-3.

**INTERNATIONAL SEARCH REPORT**

International application No.
PCT/JP2019/034972

(Invention 5) Invention including a positive surfactant in claims 1-7

The invention including a positive surfactant in claims 1-7 and the invention classified as inventions 1-4 share the common technical feature of a reagent which includes a surfactant and is for measuring hemoglobin used to measure hemoglobin by a cation exchange liquid chromatography, but said technical feature cannot be considered a special technical feature like invention 2.

In addition, there are no other identical or corresponding special technical features between these inventions.

Furthermore, the invention including a positive surfactant in claims 1-7 is not substantially identical or equivalent to any invention classified as inventions 1-4.

Therefore, the invention including a positive surfactant in claims 1-7 cannot be classified as inventions 1-4.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 58191968 A
- JP 2001021555 A